# EUROPEAN PATENT SPECIFICATION

(11) **EP 2 111 228 B1**
(45) Date of publication and mention of the grant of the patent: **20.07.2011**
(21) Application number: 08725121.1
(22) Date of filing: 01.02.2008
(51) Int. Cl.: A61K 38/39, A61P 9/10, A61P 35/00, A61P 37/00, A61P 29/00

(54) **10Fn3 domain for use in treating diseases associated with inappropriate angiogenesis**
10Fn3 Domain zur Behandlung von Krankheiten begleitet von unerwünschter Angiogenese
Domaine 10Fn3 pour utilisation dans le traitement de maladies associées à une angiogenese inappropriée

(30) Priority: 02.02.2007 US 899094 P; 02.08.2007 US 963208 P
(43) Date of publication of application: 28.10.2009
(73) Proprietor: Bristol-Myers Squibb Company, Princeton, NJ 08543-4000 (US)
(72) Inventor: MENDLEIN, John, Scituate, MA 02066 (US); FURFINE, Eric, Concord, MA 01742 (US)
(74) Representative: Reitstötter - Kinzebach
(86) International application number: PCT/US2008/001432
(87) International publication number: WO 2008/097497

(56) References cited:
- WO-A-2005/056764

## Description

### FIELD OF THE INVENTION

The present invention relates to innovative proteins that block the VEGF-VEGFR pathway mediated biology and pathology for use in treating certain conditions associated with inappropriate angiogenesis.

### INTRODUCTION

Angiogenesis is the process by which new blood vessels are formed from pre-existing capillaries or post capillary venules; it is an important component of many physiological processes including ovulation, embryonic development, wound repair, and collateral vascular generation in the myocardium. Angiogenesis is also central to a number of pathological conditions such as tumor growth and metastasis, diabetic retinopathy, and macular degeneration. In many instances, the process begins with the activation of existing vascular endothelial cells in response to a variety of cytokines and growth factors. In cancer, tumor released cytokines or angiogenic factors stimulate vascular endothelial cells by interacting with specific cell surface receptors. The activated endothelial cells secrete enzymes that degrade the basement membrane of the vessels, allowing invasion of the endothelial cells into the tumor tissue. Once situated, the endothelial cells differentiate to form new vessel offshoots of pre-existing vessels. The new blood vessels provide nutrients to the tumor, facilitating further growth, and also provide a route for metastasis.

To date, numerous angiogenic factors have been identified, including the particularly potent factor VEGF. VEGF was initially purified from the conditioned media of folliculostellate cells and from a variety of cell lines. More recently a number of structural homologs and alternatively spliced forms of VEGF have been identified. The various forms of VEGF bind as high affinity ligands to a suite of VEGF receptors (VEGFRs). VEGFRs are tyrosine kinase receptors, many of which are important regulators of angiogenesis. The VEGFR family includes 3 major subtypes: VEGFR-1, VEGFR-2 (also known as Kinase Insert Domain Receptor, "KDR", in humans), and VEGFR-3. Among VEGF forms, VEGF-A, VEGF-C and VEGF-D are known to bind and activate VEGFR-2.

VEGF, acting through its cognate receptors, can function as an endothelial specific mitogen during angiogenesis. In addition, there is substantial evidence that VEGF and VEGFRs are up-regulated in conditions characterized by inappropriate angiogenesis, such as cancer. As a result, a great deal of research has focused on the identification of therapeutics that target and inhibit VEGF or VEGFR.

In view of the roles that VEGF and VEGFR have in disorders it would be desirable to generate improved therapeutics and formulations to treat conditions associated with inappropriate angiogenesis.

WO 2005/056764 describes VEGFR-binding polypeptides comprising a tenth fibronectin type III (¹⁰Fn3) domain and their use in a condition characterized by inappropriate angiogenesis.

### SUMMARY OF THE INVENTION

The present invention relates to a polypeptide for use in treating a condition selected from an autoimmune disorder, an inflammatory disorder, a retinopathy, and a cancer, wherein the polypeptide comprises the amino acid sequence set forth in SEQ ID NO:4 and binds human VEGFR2 with a dissociation constant of 1 µM or less, wherein administration of the polypeptide results in a peak concentration of between 1 and 7 µM of the polypeptide.

In some embodiments, the polypeptide is intravenously administered prior to subcutaneous administration. For example, a first intravenous loading dose is administered followed by subcutaneous administration therapy.

In some embodiments, less than 2 ml of polypeptide is administered in a single dose. In some embodiments, less than 1 ml of polypeptide is administered in a single dose.

In some embodiments, the polypeptide is administered intravenously at least once, twice, or three times per week. In some embodiments, the polypeptide is administered intravenously once per week. In some embodiments, the polypeptide is administered intravenously once every other week. In some embodiments, the polypeptide is administered intravenously less than once every other week. In some embodiments, the polypeptide is administered subcutaneously at least once, twice, or three times per week. In some embodiments, the polypeptide is administered subcutaneously less than once, twice, or three times per week. In some embodiments, the polypeptide is administered subcutaneously at least once, twice, or three times per day. In some embodiments, the polypeptide is administered subcutaneously twice per day. In some embodiments, the polypeptide is administered subcutaneously less than once per day.

The application describes methods of determining an effective dosage of a polypeptide for administration to a subject having a condition associated with inappropriate angiogenesis. The method comprises first determining the baseline plasma concentration of VEGF-A, administering a dosage of the polypeptide to the subject, determining the plasma concentration of VEGF-A in the subject after the administration, and then adjusting the dosage of the polypeptide to achieve at least a 20% increase in the plasma concentration of VEGF-A in the subject relative to the baseline plasma VEGF-A concentration.

In some embodiments, the plasma VEGF-A concentration is determined at least 2, 4, 6, 8, 12, or 24 hours after administration of the polypeptide. In some embodiments, the plasma VEGF-A concentration determined is the peak concentration. In some embodiments, the dosage is adjusted to achieve at least a 20, 30, 40, 50, 60, 70, 80, 90% or more increase in plasma VEGF-A concentration relative to the baseline plasma concentration of VEGF-A. In some embodiments, the dosage is adjusted to achieve a maximal increase in VEGF-A plasma concentration relative to the baseline.

In some embodiments, the baseline plasma VEGF-A concentration is determined by measuring the plasma VEGF-A concentration in a subject prior to the onset of polypeptide therapy, i.e., before the subject has ever been administered a polypeptide of the invention. In some embodiments, the baseline concentration is determined by measuring the plasma VEGF-A concentration in a subject undergoing polypeptide therapy prior to administration of the polypeptide, e.g., when the polypeptide has reached its trough level. In some embodiments, the baseline concentration is determined based on the average plasma VEGF-A concentration in a population.

In the above described methods, the polypeptide comprises a tenth fibronectin type III (¹⁰Fn3) domain, wherein the ¹⁰Fn3 domain (i) comprises a loop, AB; a loop, BC; a loop, CD; a loop, DE; a loop EF; and a loop FG; (ii) has at least one loop selected from loop BC, DE, and FG with an altered amino acid sequence relative to the sequence of the corresponding loop of the human ¹⁰Fn3 domain, and (iii) binds human VEGFR2 with a disassociation constant of 1 µM or less. The polypeptide comprises an amino acid sequence selected from SEQ ID NO: 4 or SEQ ID NO: 5.

The application describes methods for determining the efficacy of a polypeptide in treating a subject having a condition associated with inappropriate angiogenesis, comprising determining the baseline plasma concentration of VEGF-A, administering a dosage of the polypeptide to the subject, determining the plasma concentration of VEGF-A in the subject after administration, and determining the efficacy of the polypeptide based on the change of the baseline plasma VEGF-A concentration compared to the plasma concentration of VEGF-A determined after polypeptide administration.

In some embodiments, the plasma VEGF-A concentration is determined at least 2, 4, 6, 8, 12, or 24 hours after administration of the polypeptide. In some embodiments, the plasma VEGF-A concentration determined is the peak concentration.

In some embodiments, at least a 20, 30, 40, 50, 60, 70, 80, 90% or more increase in plasma VEGF-A concentration after polypeptide administration compared to the baseline VEGF-A concentration indicates the efficacy of the polypeptide in treating a subject having a condition associated with inappropriate angiogenesis.

In the above described method, the polypeptide comprises a tenth fibronectin type III (¹⁰Fn3) domain, wherein the ¹⁰Fn3 domain (i) comprises a loop, AB; a loop, BC; a loop, CD; a loop, DE; a loop EF; and a loop FG; (ii) has at least one loop selected from loop BC, DE, and FG with an altered amino acid sequence relative to the sequence of the corresponding loop of the human ¹⁰Fn3 domain, and (iii) binds human VEGFR2 with a disassociation constant of 1 µM or less. The polypeptide comprises an amino acid sequence selected from SEQ ID NO: 4 or SEQ ID NO: 5.

The application describes methods for monitoring an immunogenic response to a polypeptide administered to a subject, comprising administering a dosage of the polypeptide to the subject, determining the plasma concentration of VEGF-A in the subject after administration, and comparing the plasma concentration of VEGF-A determined in the subject after administration to a VEGF-A plasma concentration determined in the subject at one or more prior time points. A decrease in the plasma concentration of VEGF-A determined after administration relative to the concentration determined at one or more prior time points indicates an immunogenic response to the polypeptide.

In some embodiments, the prior time point is before the subject began polypeptide therapy, i.e., prior to the subject ever having been administered the polypeptide. In some embodiments, the prior time point is one, two, three, four, five, or more months prior. In some embodiments, the VEGF-A plasma concentration determined at one or more prior time points is an average of the VEGF-A plasma concentration determined at two or more prior time points.

In some embodiments, the plasma VEGF-A concentration determined after polypeptide administration is determined at least 2, 4, 6, 8, 12, or 24 hours after administration of the polypeptide. In some embodiments, the plasma VEGF-A concentration determined is the peak concentration.

In some embodiments, at least a 20, 30, 40, 50, 60, 70, 80, 90% or more decrease in plasma VEGF-A concentration after polypeptide administration compared to the VEGF-A plasma concentration determined at one or more prior time points indicates an immunogenic response the polypeptide. In some embodiments, the polypeptide therapy is discontinued upon indication of an immunogenic response.

The subject has a condition associated with inappropriate angiogenesis.

In the above described method, the polypeptide comprises a tenth fibronectin type III (¹⁰Fn3) domain, wherein the ¹⁰Fn3 domain (i) comprises a loop, AB; a loop, BC; a loop, CD; a loop, DE; a loop EF; and a loop FG; (ii) has at least one loop selected from loop BC, DE, and FG with an altered amino acid sequence relative to the sequence of the corresponding loop of the human ¹⁰Fn3 domain, and (iii) binds human VEGFR2 with a disassociation constant of 1 µM or less. The polypeptide comprises an amino acid sequence selected from SEQ ID NO: 4 or SEQ ID NO: 5.

The application describes methods for monitoring the risk of toxicity of a polypeptide administered to a subject, comprising administering a dosage of the polypeptide to the subject, determining the plasma concentration of VEGF-A in the subject after administration, and comparing the plasma concentration of VEGF-A determined in the subject after administration to a VEGF-A toxicity concentration. A plasma concentration of VEGF-A determined after administration of the polypeptide that that is higher than the VEGF-A toxicity concentration indicates a risk of toxicity from the polypeptide. In some embodiments, the polypeptide therapy is discontinued upon indication of a risk of toxicity.

In some embodiments, the plasma VEGF-A concentration determined after polypeptide administration is determined at least 2, 4, 6, 8, 12, or 24 hours after administration of the polypeptide. In some embodiments, the plasma VEGF-A concentration determined is the peak concentration.

In some embodiments, the toxicity risk concentration is a plasma concentration of VEGF-A of at least 2, 3, 4, 5, 6, 7, 8, 9, 10, 15, 20, 30, 40, 50 pM or more. In some embodiments, the toxicity risk concentration is determined from the subject's baseline VEGF-A plasma concentration, i.e., the VEGF-A plasma concentration in a subject determined prior to the onset of polypeptide therapy. The toxicity risk concentration is at least 2, 3, 4, 5, 6, 7, 8, 9, 10, 15, 20, 30 times or more the concentration of the subject's baseline VEGF-A plasma concentration. In some embodiments, the VEGF-A toxicity risk concentration is determined based on the average VEGF-A toxicity risk concentration in a population.

In some embodiments, the subject has a condition associated with inappropriate angiogenesis.

In the above described method, the polypeptide comprises a tenth fibronectin type III (¹⁰Fn3) domain, wherein the ¹⁰Fn3 domain (i) comprises a loop, AB; a loop, BC; a loop, CD; a loop, DE; a loop EF; and a loop FG; (ii) has at least one loop selected from loop BC, DE, and FG with an altered amino acid sequence relative to the sequence of the corresponding loop of the human ¹⁰Fn3 domain, and (iii) binds human VEGFR2 with a disassociation constant of 1 µM or less. The polypeptide comprises an amino acid sequence selected from SEQ ID NO: 4 or SEQ ID NO: 5.

According to the invention, the subject has a condition associated with inappropriate angiogenesis selected from an autoimmune disorder, an inflammatory disorder, a retinopathy, and a cancer.

In some embodiments of the methods described herein, the polypeptide is administered as a formulation comprising 1 to 15 mg/kg of the polypeptide, 5 to 100 mM sodium acetate, 0 to 200 mM sodium chloride, and 50 to 150 mM mannitol, wherein the pH of the formulation is from 4.5 to 6.0. The formulation may also be diluted prior to administration.

The polypeptides of the invention bind VEGFR2 with high affinity. In some embodiments, the polypeptides bind VEGFR2 with a disassociation constant of about 1 µM or less, about 10 nM or less, or about 1 nM or less. In some embodiments, the polypeptide of the invention inhibits the binding of VEGF-A, VEGF-C and/or VEGF-D to VEGFR2 and does not activate human VEGFR2 at sub IC50 concentrations in a cell-based assay with an IC50 of less than about 1 nM or about 100 pM. In some embodiments, the polypeptide of the invention induces apoptosis in a cell based assay in a cell line dependent on VEGFR2 activation. In some embodiments, VEGFR2 binders block VEGFR2 activities such as control of apoptosis, phosphorylation or dimerization.

It will be often desirable, particularly in vivo applications, that proteins of the invention targeting VEGFR2 either as a mono-specific therapeutic or multi-specific (including bi-specific or tri-specific) therapeutics are selective over VEGFR2 compared to VEGFR1 or VEGFR3. Such selectivity is preferably at least about 100 times, at least about 1000 times, at least about 10,000 times and at least about 100,000 times. In addition, it my be desirable, particularly for proteins with high affinity to VEGFR2 to not detectably bind VEGFR1 or VEGFR3 at a defined concentration or lower of therapeutic or protein, such concentrations are about 100 nM, about 1 uM, or about 10 uM. Selectivity relationships of proteins that bind to VEGFR2 over VEGFR1 or VEGFR3 may also be expressed by comparing Kd, IC50, and Ki's either as measured or calculated depending on the assay; or as a ratio of the same biochemical or biological parameters (e.g., Kd, IC50, and Ki's). Such ratios preferably include ratios of VEGFR1 or VEGFR3 to VEGFR2 binding of about 100, about 1,000, about 10,000 or about 100,000. Other proteins of the invention that bind to other targets, particularly tyrosine kinase receptors, preferably are selective for the desired target (e.g., EGFR or Her2) compared to a closely related target using the selectivity guidance provided herein.

The polypeptide comprises a fibronectin-based scaffold protein. In some embodiments, the polypeptide comprising a tenth fibronectin type III (¹⁰Fn3) domain, wherein the ¹⁰Fn3 domain (i) comprises a loop, AB; a loop, BC; a loop, CD; a loop, DE; a loop EF; and a loop FG; (ii) has at least one loop selected from loop BC, DE, and FG with an altered amino acid sequence relative to the sequence of the corresponding loop of the human ¹⁰Fn3 domain, and (iii) binds human VEGFR2 with a disassociation constant of about 1 µM or less. Loop BC and loop FG have an altered amino acid sequence relative to the sequence of the corresponding loop of the human ¹⁰Fn3 domain. Substantially monovalent binding to VEGFR2 via fibronectin based scaffolds is helpful in reducing VEGFR2 activation in this and other embodiments of the invention.

In some embodiments, the polypeptides of the invention further comprise one or more pharmacokinetic (PK) moieties. PK moieties improve one or more pharmacokinetic properties of the polypeptides, e.g., bioavailability, serum half-life, in vivo stability, and drug distribution. In some embodiments, the PK moiety is selected from: a polyoxyalkylene moiety, a human serum albumin binding protein, sialic acid, human serum albumin, transferrin, and Fc or an Fc fragment. In some embodiments, the PK moiety is polyethylene glycol (PEG). In some embodiments, the PEG moiety is covalently linked to the polypeptide of the invention via a Cys or Lys amino acid. In some embodiments, a polypeptide of the invention is linked by at least one peptide bond to a second protein that binds human serum albumin (HSA) with a binding affinity of about 300 nM or less.

In some embodiments, the polypeptides of the invention further comprise a second domain that binds to a human protein. In some embodiments the human protein is selected from, IGF-IR, EGFR, folate receptor, Her2, Her3, c-kit, c-Met, FGFR1, FGFR2, PDGFR, VEGFR1, VEGFR2, VEGF3, Tie2, Ang1, Ang2, FGF (fibroblast growth factor), EGF (epidermal growth factor), HGF (hepatocyte growth factor), VEGF-A (vascular endothelial growth factor - A), VEGF-C, and VEGF-D. In some embodiments, the human protein is a tyrosine receptor. In some embodiments, the second domain binds to a human protein with a disassociation constant of about 1 µM or less, about 10 nM or less, or about 1 nM or less.

In some embodiments, the polypeptide comprises a first and second protein, wherein either or both proteins are a single chain antibody and the first and second proteins are linked via a PEG moiety. In some embodiments, the first protein binds VEGFR2.

In some embodiments, the second domain is selected from: an antibody moiety of less than about 50 kDa; a derivative of lipocalin; a derivative of tetranectin; an avimer; a derivative of ankyrin, and a second tenth fibronectin type III (¹⁰Fn3) domain, wherein the second ¹⁰Fn3 domain comprises a loop, AB; a loop, BC; a loop, CD; a loop, DE; and a loop FG; and has at least one loop selected from loop BC, DE, and FG with a randomized amino acid sequence relative to the sequence of the corresponding loop of the human ¹⁰Fn3 domain, and binds a human protein, which is not bound by the human ¹⁰Fn3 domain, with a disassociation constant of about 10 nM or less. In some embodiments, the polypeptide of the invention and the second domain are operably linked via at least one disulfide bond, a peptide bond, a polypeptide, a polymeric sugar, or a polyethylene glycol (PEG) moiety. In some embodiments, the PEG is between about 0.5 kDa and about 100 kDa.

In some embodiments of the invention, polypeptides of the invention comprise a first and second protein linked via a polypeptide, wherein the first or second protein or polypeptide linker has a protease site that is cleavable by a protease in the blood or target tissue. Such embodiments can be used to release two or more therapeutic proteins for better delivery or therapeutic properties or more efficient production compared to separately producing such proteins.

In some embodiments, a first and second protein are linked using a biocompatible polymer such as a polymeric sugar. Such polymeric sugar can include an enzymatic cleavage site that is cleavable by an enzyme in the blood or target tissue. Such embodiments can be used to release two or more therapeutic proteins for better delivery or therapeutic properties or more efficient production compared to separately producing such proteins.

Another aspect of the invention is the operable linkage of one or more proteins of the invention, or other proteins described herein to make a protein therapeutic of the invention, via a polymer to create multi-functional proteins. For instance the polymer could be a polypeptide, polymeric sugar, carbon based polymer (e.g., aliphatic chain) or PEG. An additional aspect of the invention includes linking a protein of the invention that binds VEGFR2 as described herein with PEG to another protein, such as: a Fab, a single chain Ab, domain antibodies, camel antibodies and their derivatives (particularly entities less than about 50 kDa), antibody moiety (e.g., less than about 50 kDa), an Adtein (e.g., a protein between 5 and 40 kDa the binds a target with a desired specificity while having one or more of pharmaceutical or biochemical properties described herein, particularly those related to tyrosine kinase receptors), a fibronectin based scaffold protein (e.g., an Adnectin^{™}), or antibody alternatives, such as Avimers, microbodies, Trans-bodies^{™}, Affibodies^{™}, Affilins^{™}, or trinectins (e.g., tetranectins) and other derivatives of proteins like lipocalin, or ankyrin (DARPin). In some embodiments, the first and second linked proteins collectively have 1 or 0 disulfide bonds. This may be desirable to improve protein production in cell based systems. Preferably, said first protein is substantially a single domain that has substantially monovalent binding to VEGFR2. This may be desirable to improve protein production in cell based systems. Preferably, said first protein binds human VEGFR2 with an binding affinity of about 100 pM or less and has at least two structural loops that participate in the binding of said first protein to human VEGFR2. Preferably, said second protein is a fibronectin based scaffold protein linked by at least one peptide bond to said first protein. In some embodiments, said first protein and second protein are linked by at least one disulfide bond. Though this can be accomplished in cells in may be desired to perform this linkage *in vitro* without cells. The polypeptide of the invention may include a first protein that binds human VEGFR2 with an binding affinity of about 300 pM or less and has at least two structural loops that participate in the binding of said first protein to human VEGFR2. Preferably, said first protein binds human VEGFR2 with an binding affinity of about 1 nM or less and binds human insulin receptor with a binding affinity of about 1µM or greater. Preferably, the polypeptide of the invention further comprises a PEG moiety operably linked either said first protein or said second protein. Preferably, said second protein binds a human tyrosine kinase receptor up-regulated in a human cancer, wherein said second protein binds the human tyrosine kinase receptor with an binding affinity of about 10 nM or less and binds human insulin receptor with a binding affinity of about 1 uM or greater.

In some embodiments, the polypeptide of the invention may comprise a first protein and said second protein that collectively have at least 6 disulfide bonds or, in another aspect, collectively have at least 8 disulfide bonds. Preferably, said first protein and said second protein are a single polypeptide expressed from a microbe. Preferably, said first protein binds human VEGFR2 with a binding affinity of about 100 pM or less and has at least two structural loops that participate in the binding of said first protein to human VEGFR2. In some embodiments, at least one of said first protein and said second protein is an antibody moiety. Preferably, such antibody moiety is less than about 50 kDa or, in another aspect, is less than about 40 kDa. In some embodiments, the antibody moiety is a single chain antibody moiety. The protein therapeutic includes an embodiment wherein said second protein binds one of the following human proteins: EGFR, folate receptor, Her2, Her3, c-kit, c-Met, FGFR1, FGFR2, PDGFR, VEGFR1, VEGFR2, VEGF3, and Tie2; and ligands: Ang1, Ang2, FGF, EGF, HGF, stem cell factor (SCF), VEGF-A, VEGF-C, and VEGF-D. In some embodiments, the first protein and/or the second protein are a derivative of lipocalin. In some embodiments, least one of said first protein and said second protein is a derivative of a tetranectin. In some embodiments, at least one of said first protein moiety and said second protein moiety is a derivative of an avimer.

PEG may be used in many aspects of the invention and different sizes may be used as described herein for the desired therapeutic or other *in vivo* effect, such as imaging. Larger PEGs are preferred to increase half life in the body, blood, non-blood extracellular fluids or tissues. For *in vivo* cellular activity, PEGs of the range of about 10 to 60 kDa are preferred, as well as PEGs less than about 100 kDa and more preferably less than about 60 kDa, though sizes greater than about 100 kDa can be used as well. For *in vivo* imaging application, smaller PEGs, generally less than about 20 kDa, may be used that do not increase half life as much as larger PEGs so as to permit quicker distribution and less half life.

In some embodiments, polypeptides of the invention comprise a first protein and second protein operably linked to PEG through a single Cys or Lys. In some embodiments, at least the first or second protein has no more than a single Cys or Lys. In some embodiments, the single Cys or Lys is located in said first or second protein in a non-wildtype location in the amino acid sequence.

In some embodiments, the polypeptide of the invention comprises a first and second protein, wherein said first protein inhibits cell proliferation, has a Tm of at least 55°C, and a non-wildtype Cys or Lys in region of its amino acid sequence that does not substantially interfere with binding to human VEGFR2. In some embodiments, the first protein and second protein are a single polypeptide expressed from a microbe. In some embodiments, the first protein binds human VEGFR2 with a binding affinity of about 50 pM or less and has at least two structural loops that participate in the binding of said first protein to human VEGFR2. In some embodiments, the polypeptide of the invention has a half life *in vivo* of at least one day with IV administration. In some embodiments, the polypeptide of the invention has an exposure level at a concentration of at least about 10 times the binding affinity of said first protein to human VEGFR2 for over 24 hours in a rodent after administration. In some embodiments, the polypeptide of the invention has an exposure level at a concentration of at least about 100 times the binding affinity of said first protein to human VEGFR2 for over 24 hours in a rodent after subcutaneous administration. In some embodiments, the first or second protein is a fibronectin based scaffold protein.

A variety of affinities of the polypeptides of the invention to VEGFR2, other human proteins, or PK moieties can be can be used depending on diagnostic, therapeutic, *in vitro* or *in vivo* application. For instance, affinities with a disassociation constant similar or less than about 1 uM, about 100 nM, about 10 nM, about 1 nM, about 100 pM, about 10 pM, about 1 pM or about 100 fM are preferred, particularly for VEGFR2. I*n vivo* testing of proteins of the invention with different affinity to the desired target aids in selecting the desired affinity for *in vivo* applications. *In vitro* testing depending on the presence of other proteins and amounts of VEGFR2 may generally use proteins of the invention of different affinity compared to *in vivo* applications. Often less affinity or weaker binding can be used *in vitro.*

The polypeptides comprise fibronectin-based scaffold proteins that bind to a human target. In some embodiments, the polypeptides further comprise PK moieties as described herein. The PK moiety may be linked to the fibronectin-based scaffold protein by any suitable linker, such as those described herein.

A further aspect of the invention provides for pharmaceutically acceptable compositions comprising the polypeptides of the invention, wherein the composition is essentially endotoxin free. Preferably, the composition is substantially free of microbial contamination making it suitable for *in vivo* administration. The composition may be formulated, for example, for IV, IP or subcutaneous administration.

A further aspect of the invention provides for a cell, comprising a polynucleotide encoding one or more polypeptides of the invention. Vectors containing polynucleotides for such proteins are included as well. Sequences are preferably optimized to maximize expression in the cell type used. Preferably, expression is in E. coli. Proteins of then invention can also be expressed, for example, in eukaryotic microbes, including yeast (e.g., pichia or cervaisea) or blue green algae. Yeast cells can be engineered to produce desired glycosylations on any of the proteins described herein. The cells of the invention can be a mammalian cell. In one aspect, the mammalian cell can be engineered to produce desired glycosylations on any of the proteins described herein. In one aspect, the cell expresses a fibronectin based scaffold protein. In one aspect, the polynucleotides encoding fibronectin based scaffold proteins are codon optimized for expression in the selected cell type.

Another aspect of the invention relates to the treatment of a subject having a cancer by administering a polypeptide of the invention, either alone or in combination with other cytotoxic or therapeutic agents. In particular, preferred cytotoxic and therapeutic agents include docetaxel, paclitaxel, doxorubicin, epirubicin, cyclophosphamide, trastuzumab, capecitabine, tamoxifen, toremifene, letrozole, anastrozole, fulvestrant, exemestane, goserelin, oxaliplatin, carboplatin, cisplatin, dexamethasone, antide, bevacizumab, 5-fluorouracil, leucovorin, levamisole, irinotecan, etoposide, topotecan, gemcitabine, vinorelbine, estramustine, mitoxantrone, abarelix, zoledronate, streptozocin, rituximab, idarubicin, busulfan, chlorambucil, fludarabine, imatinib, cytarabine, ibritumomab, tositumomab, interferon alpha-2b, melphalam, bortezomib, altretamine, asparaginase, gefitinib, erlonitib, anti-EGF receptor antibody (e.g., cetuximab or panitumumab), ixabepilone, and an epothilone or derivative thereof. More preferably, the therapeutic agent is a platinum agent (such as carboplatin, oxaliplatin, cisplatin), a taxane (such as paclitaxel, docetaxel), gemcitabine, or camptothecin.

In addition, it may be preferred to combine a polypeptide of the invention with a second therapeutic protein as a single molecule or perhaps as a single molecule with a third therapeutic protein. Such a therapeutic entity comprises a protein of the invention linked by PEG or other polymer (e.g., Cys-Cys disulfide or polypeptide) to one or more therapeutic proteins. Such therapeutic proteins include antibody derivatives (e.g., Fabs, camel antibodies and their derivatives, domain antibodies (e.g., less than about 50 kDa in size) and single chains (preferably less than about 50 kDa in size)), Adnectins^{™} and proteins preferably in the range of ~5 to ~40 kDa.

Targets of proteins of the invention include, particularly human versions, although in some instances model species such as mouse, rat, monkey and dog: IGF-IR, FGFR1, FGFR2, FGFR3, FGFR4, c-Kit, human p185 receptor-like tyrosine kinase (HER2 or Her2), Her3, c-Met, folate receptor, PDGFR, VEGFR1, VEGFR2, VEGFR3, VEGF A, VEGF C, VEGF D, human CD20, human CD18, human CD11a, human apoptosis receptor-2 (Apo-2), human .alpha.4.beta.7 integrin, human GPIIb-IIIa integrin, stem cell factor (SCF), human epidermal growth factor receptor (EGFR), and human CD3. In addition, aspects of the invention include multifunctional proteins that bind a first target and at least one other target. Preferably, such proteins are linked by the PEG related inventions described herein, although in many embodiments such proteins may be linked by polypeptides or other polymeric linkers or non-polymeric linkers.

Stably linked proteins of the invention may be of use for therapeutic treatment of cancer. Multispecific proteins of the invention have the advantage of modulating, blocking or inhibiting more than one therapeutic target when directed to 2, 3, 4 or more therapeutic targets or epitopes.

It is anticipated that any type of tumor and any type of tumor antigen may be targeted with the corresponding biology of the therapeutic. The cancer can be one or more of, for example, breast cancer, colon cancer, ovarian carcinoma, osteosarcoma, cervical cancer, prostate cancer, lung cancer, synovial carcinoma, pancreatic cancer, melanoma, multiple myeloma, neuroblastoma, and rhabdomyosarcoma, or other cancer yet to be determined in which VEGFR2 levels are elevated, up-regulated, mutated or altered in physiology compared to non-oncogenic cells.

Other exemplary types of tumors that may be targeted include acute lymphoblastic leukemia, acute myelogenous leukemia, biliary cancer, breast cancer, cervical cancer, chronic lymphocytic leukemia, chronic myelogenous leukemia, colorectal cancer, endometrial cancer, esophageal, gastric, head and neck cancer, Hodgkin's lymphoma, lung cancer, medullary thyroid cancer, non-Hodgkin's lymphoma, multiple myeloma, renal cancer, ovarian cancer, pancreatic cancer, glioma, melanoma, liver cancer, prostate cancer, and urinary bladder cancer.

Additionally, tumor-associated targets may be targeted by polypeptides of the invention. In some embodiments antigen targeting will help localize the therapeutic in terms of tissue distribution or increased local concentration affect either in the tissue or desired cell type. Alternatively, it may provide an additional mechanism of action to combat cancer along with one of the targets described herein for which a therapeutic is made. Such antigens or targets include, but are not limited to, carbonic anhydrase IX, A3, antigen specific for A33 antibody, BrE3-antigen, CD1, CD1a, CD3, CD5, CD15, CD16, CD19, CD20, CD21, CD22, CD23, CD25, CD30, CD45, CD74, CD79a, CD80, HLA-DR, NCA 95, NCA90, HCG and its subunits, CEA (CEACAM-5), CEACAM-6, CSAp, EGFR, EGP-1, EGP-2, Ep-CAM, Ba 733, HER2/neu, hypoxia inducible factor (HIF), KC4-antigen, KS-1-antigen, KS1-4, Le-Y, macrophage inhibition factor (MIF), MAGE, MUC1, MUC2, MUC3, MUC4, PAM-4-antigen, PSA, PSMA, RS5, S100, TAG-72, p53, tenascin, IL-6, IL-8, insulin growth factor-I (IGF-1), insulin growth factor-II (IGF-II), Tn antigen, Thomson-Friedenreich antigens, tumor necrosis antigens, placenta growth factor (P1GF), 17-1A-antigen, an angiogenesis marker (e.g., ED-B fibronectin), an oncogene marker, an oncogene product, and other tumor-associated antigens. Recent reports on tumor associated antigens include Mizukami et al., (2005, Nature Med. 11:992-97); Hatfield et al., (2005, Curr. Cancer Drug Targets 5:229-48); Vallbohmer et al. (2005, J. Clin. Oncol. 23:3536-44); and Ren et al. (2005, Ann. Surg. 242:55-63).

In other embodiments, an anti-angiogenic agent may form a portion of a therapeutic and may be operably linked to a protein of the invention. Exemplary anti-angiogenic agents of use include angiostatin, baculostatin, canstatin, maspin, anti-VEGF antibodies or peptides, antiplacental growth factor antibodies or peptides, anti-Flk-1 antibodies, anti-Flt-1 antibodies or peptides, laminin peptides, fibronectin peptides, plasminogen activator inhibitors, tissue metalloproteinase inhibitors, interferons, interleukin 12, IP-10, Gro-.beta., thrombospondin, 2-methoxyoestradiol, proliferin-related protein, carboxiamidotriazole, CM101, Marimastat, pentosan polysulphate, angiopoietin 2, interferon-alpha, herbimycin A, PNU145156E, 16K prolactin fragment, Linomide, thalidomide, pentoxifylline, genistein, TNP-470, endostatin, paclitaxel, accutin, angiostatin, cidofovir, vincristine, bleomycin, AGM-1470, platelet factor 4 or minocycline.

Another aspect of the invention provides kits comprising one or more of the elements described herein, and instructions for the use of those elements. In a preferred embodiment, a kit of the present invention includes a protein of the invention, alone or with a second therapeutic agent. The instructions for this preferred embodiment include instructions for inhibiting the growth of a cancer cell using a protein of the invention, alone or with a second therapeutic agent, and/or instructions for a method of treating a patient having a cancer using the same.

### BRIEF DESCRIPTION OF THE DRAWINGS

Figure 1 depicts the Comp-I blocking effect of VEGFR2 activation in a Murine Pro-B Cell line Proliferation Assay. X-axis denotes the concentration of Comp-I (nM); y-axis denotes OD (490).
Figure 2 demonstrates the effect of vehicle (-◆-), 30 mg/kg Comp-I (-▲-), or 5 mg/kg bevacizumab (-×-) bi-weekly treatment on tumor volume in the U87 xenograft model over the course of 20 days. Days from initial treatment are plotted on the X-axis; tumor volume (mm³) is plotted on the y-axis.
Figure 3 demonstrates the effect of vehicle (D), 60 mg/kg Comp-I (A), 60 mg/kg sunitinib (B), and 60 mg/kg sorafenib (C) treatment of tumor size in a Colo205 colon carcinoma model. The percentage of mice with tumors less than 500 mm³ is plotted on the y-axis; number of days following treatment on the x-axis.
Figure 4 demonstrates the effect of Comp-I on microvascular density in a U87 human glioblastoma mouse xenograft model. The rat anti-mouse VEGFR2 monoclonal antibody DC101 (ATCC) was also tested. Microvascular density (mm²)⁻¹ is plotted on the y-axis. Comp-I was administered three times weekly at 3, 10, and 30 mg/kg. DC101 was administered at 40mg/kg, twice a week.
Figure 5 depicts the increase in blood pressure observed after treatment of rats with Comp-I.
Time in hours is plotted on the x-axis; mean arterial pressure (mm Hg) is plotted on the y-axis.
The first arrow along the x-axis indicates administration of vehicle, while the second arrow indicates administration of Comp-I. *p < 0.05; ** p < 0.005
Figure 6 demonstrates that the first dose pharmokinetic profile is similar among subjects of the phase I trial. Comp-I concentrations were measured with an enzyme-linked immunosorbent assay (ELISA). Comp-I (µM) is plotted on the y-axis; time in weeks in plotted on the x-axis.
Figure 7 demonstrates that the pharmokinetic profile is similar after 6 months of weekly 1 mg/kg Comp-I treatment. Results are shown for subject 1003. Comp-I (µM) is plotted on the y-axis;
time in weeks in plotted on the x-axis. PK profile of first treatment is marked by triangle; PK profile after 6 months is marked by square.
Figure 8 demonstrates that trough levels of Comp-I are consistent over time. The percent change of Comp-I concentration from the first trough is plotted on the y-axis as an average of the two patient cohorts.
Figures 9A and 9B depict the percentage of animals surviving with tumors smaller than 300 mm³ (Figure 9A) and 1000 mm³ (Figure 9B) with Comp-I treatment three times per week.
Figure 10 depicts Comp-I induced VEGF-A levels in mice. N=3 for all groups. Error bars denote the mean - SD.
Figure 11 demonstrates that pre-infusion plasma VEGF-A levels are greater than 70% of the four hour post-infusion (peak) levels following one course of weekly treatment. VEGF-A (pM) concentration is plotted on the y-axis.
Figure 12 demonstrates the effect of Comp-I treatment on VEGF-A plasma levels. Results from subject 1002 with 1mg/kg dosage of Comp-I. First arrow indicates initial Comp-I treatment;
second arrow indicates time of second treatment. VEGF-A (pM) concentration is plotted on the y-axis.
Figure 13 shows that the plasma soluble VEGFR2 levels are maintained significantly above baseline over time after treatment. The concentration of soluble VEGFR2 (pM) in plasma (x-axis) from three subjects is plotted against the number of days after initial treatment (y-axis).
Figure 14 depicts the average pharmacokinetics of Comp-I for the 6 subjects treated weekly with 1 mg/kg (circles) and the 6 subjects treated weekly with 3 mg/kg (squares). The number of days from administration is plotted on the x-axis; concentration of Comp-I (µM) is plotted on the y-axis.
Figure 15 depicts a schematic of a Berkley Madonna model defined as a subcutaneous compartment that distributes with a first order process (k1) to a systemic compartment where the drug is subsequently eliminated by a first order process (k2).
Figure 16 depicts pharmacokinetic modeling of Comp-I. Days are plotted on the x-axis; plasma concentration of Comp-I (µM) plotted on the y-axis. The predicted pharmacokinetic profile is plotted for 0.1 mg/kg, once per day subcutaneous dosing of Comp-I (marked as "A"); 0.1 mg/kg, once per day subcutaneous dosing of Comp-I with an initial 1 mg/kg intravenous loading Comp-I dose (marked as "B"); and 1 mg/kg, once weekly intraveneous Comp-I administration.

### DETAILED DESCRIPTION OF THE INVENTION

### Definitions

By a "polypeptide" is meant any sequence of two or more amino acids, regardless of length, post-translation modification, or function. "Polypeptide," "peptide," and "protein" are used interchangeably herein. Polypeptides can include natural amino acids and non-natural amino acids such as those described in U.S. Patent No. 6,559,126. Polypeptides can also be modified in any of a variety of standard chemical ways (e.g., an amino acid can be modified with a protecting group; the carboxy-terminal amino acid can be made into a terminal amide group; the amino-terminal residue can be modified with groups to, e.g., enhance lipophilicity; or the polypeptide can be chemically glycosylated or otherwise modified to increase stability or *in vivo* half-life). Polypeptide modifications can include the attachment of another structure such as a cyclic compound or other molecule to the polypeptide and can also include polypeptides that contain one or more amino acids in an altered configuration (i.e., R or S; or, L or D). The term "single domain polypeptide" is used to indicate that the target binding activity (e.g., VEGFR2 binding activity) of the subject polypeptide is situated within a single structural domain, as differentiated from, for example, antibodies and single chain antibodies, where antigen binding activity is generally contributed by both a heavy chain variable domain and a light chain variable domain. It is contemplated that a plurality of single domain polypeptides of the sort disclosed herein could be connected to create a composite molecule with increased avidity. Likewise, a single domain polypeptide may be attached (e.g., as a fusion protein) to any number of other polypeptides, such as fluorescent polypeptides, targeting polypeptides and polypeptides having a distinct therapeutic effect.

The term "PK" is an acronym for "pharmokinetic" and encompasses properties of a compound including, by way of example, absorbtion, distribution, metabolism, and elimination by a subject. A "PK modulation protein" or "PK moiety" refers to any protein, peptide, or moiety that affects the pharmokinetic properties of a biologically active molecule when fused to or administered together with the biologically active molecule. Examples of a PK modulation protein or PK moiety include PEG, human serum albumin (HSA) binders (as disclosed in U.S. Publication Nos. 20050287153 and 20070003549), human serum albumin, Fc or Fc fragments, and sugars (e.g., sialic acid).

Single domain polypeptides of either the immunoglobulin or immunoglobulin-like scaffold will tend to share certain structural features. For example, the polypeptide may comprise between about 80 and about 150 amino acids, which amino acids are structurally organized into a set of beta or beta-like strands, forming beta sheets, where the beta or beta-like strands are connected by intervening loop portions. The beta sheets form the stable core of the single domain polypeptides, while creating two "faces" composed of the loops that connect the beta or beta-like strands. As described herein, these loops can be varied to create customized ligand binding sites, and, with proper control, such variations can be generated without disrupting the overall stability of the protein. In antibodies, three of these loops are the well-known Complementarity Determining Regions (or "CDRs").

Scaffolds for formation of a single domain polypeptides should be highly soluble and stable in physiological conditions. Examples of immunoglobulin scaffolds are the single domain

V_{H} or V_{L} scaffold, as well as a single domain camelid V_{HH} domain (a form of variable heavy domain found in camelids) or other immunoglobulin variable domains found in nature or engineered in the laboratory. In the single domain format disclosed herein, an immunoglobulin polypeptide need not form a dimer with a second polypeptide in order to achieve binding activity. Accordingly, any such polypeptides that naturally contain a cysteine which mediates disulfide cross-linking to a second protein can be altered to eliminate the cysteine. Alternatively, the cysteine may be retained for use in conjugating additional moieties, such as PEG, to the single domain polypeptide.

Other scaffolds may be non-antibody scaffold proteins. By "non-antibody scaffold protein or domain" is meant a non-antibody polypeptide having an immunoglobulin-like fold. By "immunoglobulin-like fold" is meant a protein domain of between about 80-150 amino acid residues that includes two layers of antiparallel beta-sheets, and in which the flat, hydrophobic faces of the two beta-sheets are packed against each other. An example of such a scaffold is the "fibronectin-based scaffold protein", by which is meant a polypeptide based on a fibronectin type III domain (Fn3). An example of fibronectin-based scaffold proteins are Adnectins^{™} (Adnexus Therapeutics, Inc.). Fibronectin is a large protein which plays essential roles in the formation of extracellular matrix and cell-cell interactions; it consists of many repeats of three types (types I, II, and III) of small domains (Baron et al., 1991). Fn3 itself is the paradigm of a large subfamily which includes portions of cell adhesion molecules, cell surface hormone and cytokine receptors, chaperoning, and carbohydrate-binding domains. For reviews see Bork & Doolittle, Proc Natl Acad Sci U S A. 1992 Oct 1;89(19):8990-4; Bork et al., J Mol Biol. 1994 Sep 30;242(4):309-20; Campbell & Spitzfaden, Structure. 1994 May 15;2(5):333-7; Harpez & Chothia, J Mol Biol. 1994 May 13;238(4):528-39).

Preferably, the fibronectin-based scaffold protein is a "10Fn3" scaffold, by which is meant a polypeptide variant based on the tenth module of the human fibronectin type III protein in which one or more of the solvent accessible loops has been randomized or mutated, particularly one or more of the three loops identified as the BC loop (amino acids 23-30), DE loop (amino acids 52-56) and FG loop (amino acids 77-87) (the numbering scheme is based on the sequence on the wild-type tenth module of the human fibronectin type III domain:

### VSDVPRDLEVVAATPTSLLISWDAPAVTVRYYRITYGETGGNSPVQEFTVPGSKSTATIS

GLKPGVDYTITGYAVTGRGDSPASSKPISINYRT (SEQ ID NO:1). Preferably, fibronectin-based scaffold proteins are based on SEQ ID NO:1.

A variety of mutant 10fen3 scaffolds have been reported. In one aspect, one or more of Asp 7, Glu 9, and Asp 23 is replaced by another amino acid, such as, for example, a non-negatively charged amino acid residue (e.g., Asn, Lys, etc.). These mutations have been reported to have the effect of promoting greater stability of the mutant 10Fn3 at neutral pH as compared to the wild-type form (See, PCT Publication No. WO02/04523) A variety of additional alterations in the 10fen3 scaffold that are either beneficial or neutral have been disclosed. See, for example, Batori et al., Protein Eng. 2002 Dec; 15(12):1015-20; Koide et al., Biochemistry 2001 Aug 28;40(34):10326-33.

Both variant and wild-type ¹⁰Fn3 proteins are characterized by the same structure, namely seven beta-strand domain sequences designated A through G and six loop regions (AB loop, BC loop, CD loop, DE loop, EF loop, and FG loop) which connect the seven beta-strand domain sequences. The beta strands positioned closest to the N- and C-termini may adopt a beta-like conformation in solution. In SEQ ID NO:1, the AB loop corresponds to residues 15-16, the BC loop corresponds to residues 22-30, the CD loop corresponds to residues 39-45, the DE loop corresponds to residues 51-55, the EF loop corresponds to residues 60-66, and the FG loop corresponds to residues 76-87. The BC loop, DE loop, and FG loop are all located at the same end of the polypeptide. Similarly, immunoglobulin scaffolds tend to have at least seven beta or beta-like strands, and often nine beta or beta-like strands. Fibronectin-based scaffold proteins can include other Fn3 type fibronectin domains as long as they exhibit useful activities and properties similar to ¹⁰Fn3 type domains.

A single domain polypeptide disclosed herein may have at least five to seven beta or beta-like strands distributed between at least two beta sheets, and at least one loop portion connecting two beta or beta-like strands, which loop portion participates in binding to VEGFR2, with the binding characterized by a dissociation constant that is less than 1x10⁻⁶M, and preferably less than 1x10⁻⁸M. As described herein, polypeptides having a dissociation constant of less than 5x10⁻⁹M are particularly desirable for therapeutic use in vivo to inhibit ligand signaling. Polypeptides having a dissociation constant of between 1x10⁻⁶M and 5x10⁻⁹M may be desirable for use in detecting or labeling, ex vivo or in vivo, VEGFR2 proteins.

Optionally, the "VEGFR2 binding protein" will bind specifically to VEGFR2 relative to other related proteins from the same species. By "specifically binds" is meant a polypeptide that recognizes and interacts with a target protein (e.g., VEGFR2) but that does not substantially recognize and interact with other molecules in a sample, for example, a biological sample. In preferred embodiments a polypeptide of the invention will specifically bind a VEGFR2 with a K_{D} at least as tight as 500 nM. Preferably, the polypeptide will specifically bind a VEGFR2 with a K_{D} of 1 pM to 500 nM, more preferably 1 pM to 100 nM, more preferably 1 pM to 10 nM, and most preferably 1 pM to 1 nM or lower.

A "functional Fc region" possesses at least one "effector function" of a native sequence Fc region. Exemplary "effector functions" include Clq binding; complement dependent cytotoxicity (CDC); Fc receptor binding; antibody-dependent cell-mediated cytotoxicity (ADCC); phagocytosis; down regulation of cell surface receptors (e.g., B cell receptor; BCR), etc. Such effector functions generally require the Fc region to be combined with a binding domain (e.g., an antibody variable domain) and can be assessed using various assays known in the art for evaluating such antibody effector functions.

A "native sequence Fc region" comprises an amino acid sequence identical to the amino acid sequence of an Fc region found in nature.

A "variant Fc region" comprises an amino acid sequence which differs from that of a native sequence Fc region by virtue of at least one amino acid modification. Preferably, the variant Fc region has at least one amino acid substitution compared to a native sequence Fc region or to the Fc region of a parent polypeptide, e.g., from about one to about ten amino acid substitutions, and preferably from about one to about five amino acid substitutions in a native sequence Fc region or in the Fc region of the parent polypeptide. The variant Fc region herein will preferably possess at least about 80% sequence identity with a native sequence Fc region and/or with an Fc region of a parent polypeptide, and most preferably at least about 90% sequence identity therewith, more preferably at least about 95% sequence identity therewith.

"Antibody-dependent cell-mediated cytotoxicity" and "ADCC" refer to a cell-mediated reaction in which nonspecific cytotoxic cells that express Fc receptors (FcRs) (e.g., Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an in vitro ADCC assay, such as that described in U.S. Patent Nos. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. PNAS (USA) 95:652-656 (1998).

"Percent (%) amino acid sequence identity" herein is defined as the percentage of amino acid residues in a candidate sequence that are identical with the amino acid residues in a selected sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are obtained as described below by using the sequence comparison computer program ALIGN-2. The ALIGN-2 sequence comparison computer program was authored by Genentech, Inc. has been filed with user documentation in the U.S. Copyright Office, Washington D.C., 20559, where it is registered under U.S. Copyright Registration No. TXU510087, and is publicly available through Genentech, Inc., South San Francisco, Calif. The ALIGN-2 program should be compiled for use on a UNIX operating system, preferably digital UNIX V4.0D. All sequence comparison parameters are set by the ALIGN-2 program and do not vary.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

A "polypeptide chain" is a polypeptide wherein each of the domains thereof is joined to other domain(s) by peptide bond(s), as opposed to non-covalent interactions or disulfide bonds.

An "isolated" polypeptide is one that has been identified and separated and/or recovered from a component of its natural environment. Contaminant components of its natural environment are materials that would interfere with diagnostic or therapeutic uses for the polypeptide, and may include enzymes, hormones, and other proteinaceous or nonproteinaceous solutes. In preferred embodiments, the polypeptide will be purified (1) to greater than 95% by weight of polypeptide as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated polypeptide includes the polypeptide in situ within recombinant cells since at least one component of the polypeptide's natural environment will not be present. Ordinarily, however, isolated polypeptide will be prepared by at least one purification step.

Targets may also be fragments of said targets. Thus a target is also a fragment of said target, capable of eliciting an immune response. A target is also a fragment of said target, capable of binding to a single domain antibody raised against the full length target.

A fragment as used herein refers to less than 100% of the sequence (e.g., 99%, 90%, 80%, 70%, 60%, 50%, 40%, 30%, 20%, 10% etc.), but comprising 5, 6, 7, 8, 9, 10, 12, 13, 14, 15, 16, 17, 18, 19, 20, 21, 22, 23, 24, 25 or more amino acids. A fragment is of sufficient length such that the interaction of interest is maintained with affinity of 1.times. 10.sup.-6M or better.

A fragment as used herein also refers to optional insertions, deletions and substitutions of one or more amino acids which do not substantially alter the ability of the target to bind to a single domain antibody raised against the wild-type target. The number of amino acid insertions deletions or substitutions is preferably up to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 or 70 amino acids.

A protein of the invention that "induces cell death" is one which causes a viable cell to become nonviable. The cell is generally one which expresses the antigen to which the protein binds, especially where the cell overexpresses the antigen. Preferably, the cell is a cancer cell, e.g., a breast, ovarian, stomach, endometrial, salivary gland, lung, kidney, colon, thyroid, pancreatic or bladder cell. In vitro, the cell may be, for example, a SKBR3, BT474, Calu 3, MDA-MB453, MDA-MB-361 or SKOV3 cell. Cell death in vitro may be determined in the absence of complement and immune effector cells to distinguish cell death induced by antibody dependent cell-mediated cytotoxicity (ADCC) or complement dependent cytotoxicity (CDC). Thus, the assay for cell death may be performed using heat inactivated serum (i.e., in the absence of complement) and in the absence of immune effector cells. To determine whether the protein of the invention is able to induce cell death, loss of membrane integrity as evaluated by uptake of propidium iodide (PI), trypan blue (see Moore et al. Cytotechnology 17:1-11 (1995)) or 7AAD can be assessed relative to untreated cells.

A protein of the invention that "induces apoptosis" is one that induces programmed cell death as determined by binding of apoptosis related molecules or events, such as annexin V, fragmentation of DNA, cell shrinkage, dilation of endoplasmic reticulum, cell fragmentation, and/or formation of membrane vesicles (called apoptotic bodies). The cell is one which expresses the antigen to which the protein binds and may be one which overexpresses the antigen. The cell may be a tumor cell, e.g. a breast, ovarian, stomach, endometrial, salivary gland, lung, kidney, colon, thyroid, pancreatic or bladder cell. In vitro, the cell may be, for example, SKBR3, BT474, Calu 3 cell, MDA-MB453, MDA-MB-361 or SKOV3 cell. Various methods are available for evaluating the cellular events associated with apoptosis. For example, phosphatidyl serine (PS) translocation can be measured by annexin binding; DNA fragmentation can be evaluated through DNA laddering as disclosed in the example herein; and nuclear/chromatin condensation along with DNA fragmentation can be evaluated by any increase in hypodiploid cells. Preferably, the protein that induces apoptosis is one which results in about 2 to 50 fold, preferably about 5 to 50 fold, and most preferably about 10 to 50 fold, induction of annexin binding relative to untreated cell in an annexin binding assay using cells expressing the antigen to which the protein of the invention binds.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the time to disease progression (TTP) and/or determining the response rates (RR).

### Overview

The application relates in part to formulations and modifications of VEGFR2 binding polypeptides as described in U.S. Publication Nos. 20070148126 and 20070160533. These binding polypeptides belong to a class of fibronectin based scaffold domain proteins and demonstrate specificity for VEGFR2 over VEGFR1. Other VEGFR inhibitors, such as bevacizumab, sunitinib, and sorafenib, affect both VEGFR1 and VEGFR2 signaling.

The present application provides novel proteins that bind VEGFR2 with advantageous properties, including, but not limited to: monovalent or multivalent binding modes (e.g., one or more than one domains that bind a particular target (including VEGFR2 and other tyrosine kinase receptors); high selectivity for the desired target over other receptors, particularly with respect to proteins binding VEGFR2 selectively over VEGFR1 or VEGFR3; tunable affinity, including a range from about 100 nM to less than about 10 pM (including femtomolar affinity); specific antagonist activity while having minimal or undetectable agonist activity; blocking of VEGFR2 ligand binding or activation; monospecific or multispecific binding to desired targets; single or multiple epitope binding; prolonged serum half life in rat; sub-cutaneous (SC) or intravenous (IV) dosing; small size (e.g. ∼5 kDa to ∼40 kDa); Tm's over about 55°C or over about 60°C; and substantially monomeric nature (e.g. single peak on size exclusion chromatography (SEC), such as about 90% of the area, about 95% of the area or about 98% of the area).

PROfusion^{™} technology was previously used to screen collections of nucleic acids encoding single domain polypeptides constructed using a scaffold based on the human fibronectin type three tenth domain (¹⁰Fn3) or constructed from the variable domains of antibody light chains. The expressed polypeptides, termed a "library" of scaffold proteins, was screened for polypeptides that could bind a target with high affinity. We isolated from this library of scaffold proteins novel single domain polypeptides that bind to VEGFR-2 and that, in some instances, inhibit VEGFR-2 biological activities. Such polypeptides may be used as independent, small peptide VEGFR-2 binding agents or may be situated in other proteins, particularly proteins that share an immunoglobulin or immunoglobulin-like fold. (For a detailed description of the RNA-protein fusion technology and fibronectin-based scaffold protein library screening methods see Szostak et al., U.S. Patent Nos.: 6,258,558; 6,261,804; 6,214,553; 6,281,344; 6,207,446; 6,518,018; PCT Publication Numbers WO 00/34784; WO 01/64942; WO 02/032925; and Roberts and Szostak, Proc Natl. Acad. Sci. 94:12297-12302, 1997.)

### VEGFR-2 Binding Proteins

VEGFR-2 binding polypeptides were generated as described in U.S. Publication Nos. 20070148126 and 20070160533 and PCT Publication No. WO05/056764. Sequences of VEGFR-2 binding ¹⁰Fn3 polypeptides useful for the invention are as follows:
SEQ ID NO:4
   GEVVAATPTSLLISWRHPHFPTRYYRITYGETGGNSPVQEFTVPLQPPTATISGLKPGVDY TITVYAVTDGRNGRLLSIPISINYRTEIDKPCQ
SEQ ID NO:5
   EVVAATPTSLLISWRHPHFPTRYYRITYGETGGNSPVQEFTVPLQPPTATISGLKPGVDYTI TVYAVTDGRNGRLLSIPISINYRT

Proteins of the invention include the disclosed amino acid sequences with deletions of the first 8 amino acids and may include additional amino acids at the N- or C- termini. For example, an additional MG sequence may be placed at the N-terminus. The M will usually be cleaved off, leaving a GEV... sequence at the N-terminus. The re-addition of the normal 8 amino acids at the N-terminus also produces a VEGFR2 binding protein with desirable properties. In some embodiments, the N-terminal methionine is cleaved off. For use in vivo, a form suitable for pegylation may be generated. In one embodiment, a C-terminal tail comprising a cysteine can be added.

### Additional Protein Embodiments

Proteins of the invention include a single domain polypeptide described herein which is generally a polypeptide that binds to a target, such as VEGFR2, and where target binding activity situated within a single structural domain, as differentiated from, for example, antibodies and single chain antibodies, where antigen binding activity is generally contributed by both a heavy chain variable domain and a light chain variable domain. The disclosure also provides larger proteins that may comprise single domain polypeptides that bind to target. For example, a plurality of single domain polypeptides may be connected to create a composite molecule with increased avidity or multivalency. Likewise, a single domain polypeptide may be attached (e.g., as a fusion protein) to any number of other polypeptides. In certain aspects a single domain polypeptide may comprise at least five to seven beta or beta-like strands distributed among at least two beta sheets, as exemplified by immunoglobulin and immunoglobulin-like domains. A beta-like strand is a string of amino acids that participates in the stabilization of a single domain polypeptide but does not necessarily adopt a beta strand conformation. Whether a beta-like strand participates in the stabilization of the protein may be assessed by deleting the string or altering the sequence of the string and analyzing whether protein stability is diminished. Stability may be assessed by, for example, thermal denaturation and renaturation studies. Preferably, a single domain polypeptide will include no more than two beta-like strands. A beta-like strand will not usually adopt an alpha-helical conformation but may adopt a random coil structure. In the context of an immunoglobulin domain or an immunoglobulin-like domain, a beta-like strand will most often occur at the position in the structure that would otherwise be occupied by the most N-terminal beta strand or the most C-terminal beta strand. An amino acid string which, if situated in the interior of a protein sequence would normally form a beta strand, may, when situated at a position closer to an N- or C-terminus, adopt a conformation that is not clearly a beta strand and is referred to herein as a beta-like strand.

In certain embodiments, the disclosure provides single domain polypeptides that bind to VEGFR2. Preferably the single domain polypeptides bind to human VEGFR2 and a model species VEGFR2. A single domain polypeptide may comprise between about 80 and about 150 amino acids that have a structural organization comprising: at least seven beta strands or beta-like strands distributed between at least two beta sheets, and at least one loop portion connecting two beta strands or beta-like strands, which loop portion participates in binding to VEGFR2. In other words a loop portion may link two beta strands, two beta-like strands or one beta strand and one beta-like strand. Typically, one or more of the loop portions will participate in VEGFR2 binding, although it is possible that one or more of the beta or beta-like strand portions will also participate in VEGFR2 binding, particularly those beta or beta-like strand portions that are situated closest to the loop portions. A single domain polypeptide may comprise a structural unit that is an immunoglobulin domain or an immunoglobulin-like domain. A single domain polypeptide may bind to any part of VEGFR2, although polypeptides that bind to an extracellular domain of a VEGFR2 are preferred. Binding may be assessed in terms of equilibrium constants (e.g., dissociation, K_{D}) and in terms of kinetic constants (e.g., on rate constant, kₒₙ and off rate constant, k_{off}). A single domain polypeptide will typically be selected to bind to VEGFR2 with a K_{D} of less than about 10⁻⁶M, or less than about 10⁻⁷M, about 5x10⁻⁸M, about 10⁻⁸M or less than about 10⁻⁹M. VEGFR2 binding polypeptides may compete for binding with one, or two or more members of the VEGF family, particularly VEGF-A, VEGF-C and VEGF-D, and may inhibit one or more VEGFR2-mediated biological events, such as proliferation of cancer cells and cancer metastasis. VEGFR2 binding polypeptides may be used for therapeutic purposes as well as for any purpose involving the detection or binding of VEGFR2. Polypeptides for therapeutic use will generally have a K_{D} of less than 5x10⁻⁸M, less than 10⁻⁸M or less than 10⁻⁹M, although higher K_{D} values may be tolerated where the k_{off} is sufficiently low or the kₒₙ is sufficiently high. In certain embodiments, a single domain polypeptide that binds to VEGFR2 will comprise a consensus or one or more sequences of the VEGFR2 binding sequences selected from the VEGFR2 binding clones described herein. Preferably, such sequence will be situated in a loop, particularly the BC, DE, and FG loops as described in PCT Publication No. WO2005/056764A2 by Chen et al.

The single domain polypeptide comprises an immunoglobulin-like domain. One, two, three or more loops of the immunoglobulin-like domain may participate in binding to VEGFR2. The immunoglobulin-like domain is a fibronectin type III (Fn3) domain. Such domain may comprise, in order from N-terminus to C-terminus, a beta or beta-like strand, A; a loop, AB; a beta or beta-like strand, B; a loop, BC; a beta or beta-like strand C; a loop CD; a beta or beta-like strand D; a loop DE; a beta or beta-like strand, E; a loop, EF; a beta or beta-like strand F; a loop FG; and a beta or beta-like strand G. Optionally, any or all of loops AB, BC, CD, DE, EF and FG may participate in VEGFR2 binding, although preferred loops are BC, DE and FG and in particular loops BC and FG. The Fn3 domain is an Fn3 domain derived from human fibronectin, particularly the 10^{th} Fn3 domain of fibronectin, referred to as ¹⁰Fn3. It should be noted that none of VEGFR2 binding polypeptides disclosed herein have an amino acid sequence that is identical to native ¹⁰Fn3; the sequence has been modified to obtain VEGFR2 binding proteins, but proteins having the basic structural features of ¹⁰Fn3, and particularly those retaining recognizable sequence homology to the native ¹⁰Fn3 are nonetheless referred to herein as "¹⁰Fn3 polypeptides". This nomenclature is similar to that found in the antibody field where, for example, a recombinant antibody V_{L} domain generated against a particular target protein may not be identical to any naturally occurring V_{L} domain but nonetheless the protein is recognizably a V_{L} protein. A ¹⁰Fn3 polypeptide may be at least 60%, 65%, 70%, 75%, 80%, 85%, or 90% identical to the human ¹⁰Fn3 domain, shown in SEQ ID NO:1. Much of the variability will generally occur in one or more of the loops. Each of the beta or beta-like strands of a ¹⁰Fn3 polypeptide may consist essentially of an amino acid sequence that is at least 80%, 85%, 90%, 95% or 100% identical to the sequence of a corresponding beta or beta-like strand of SEQ ID NO: 1, provided that such variation does not disrupt the stability of the polypeptide in physiological conditions. A ¹⁰Fn3 polypeptide may have a sequence in each of the loops AB, CD, and EF that consists essentially of an amino acid sequence that is at least 80%, 85%, 90%, 95% or 100% identical to the sequence of a corresponding loop of SEQ ID NO:1. In many instances, any or all of loops BC, DE, and FG will be poorly conserved relative to SEQ ID NO: 1. For example, all of loops BC, DE, and FG may be less than 20%, 10%, or 0% identical to their corresponding loops in SEQ ID NO:1. In some embodiments, only the BC and FG loops will be poorly conserved relative to SEQ ID NO: 1.

In certain embodiments, the disclosure provides polypeptides comprising a tenth fibronectin type III (¹⁰Fn3) domain, wherein the ¹⁰Fn3 domain comprises a loop, AB; a loop, BC; a loop, CD; a loop, DE; a loop EF; and a loop FG; and has at least one loop selected from loop BC, DE, and FG with an altered amino acid sequence relative to the sequence of the corresponding loop of the human ¹⁰Fn3 domain. By "altered" is meant one or more amino acid sequence alterations relative to a template sequence (corresponding human fibronectin domain) and includes amino acid additions, deletions, and substitutions. Altering an amino acid sequence may be accomplished through intentional, blind, or spontaneous sequence variation, generally of a nucleic acid coding sequence, and may occur by any technique, for example, PCR, error-prone PCR, or chemical DNA synthesis.

Additional sequences may be added to the N- or C-terminus. For example, an additional MG sequence may be placed at the N-terminus. The M will usually be cleaved off, leaving a GVS... sequence at the N-terminus. In some embodiments, linker sequences may be placed at the C-terminus of the ¹⁰Fn3 domain.

The disclosure provides a non-antibody polypeptide comprising a domain having an immunoglobulin-like fold that binds to VEGFR2. The non-antibody polypeptide may have a molecular weight of less than 20 kDa, or less than 15 kDa and will generally be derived (by, for example, alteration of the amino acid sequence) from a reference, or "scaffold", protein, such as an Fn3 scaffold. The non-antibody polypeptide may bind VEGFR2 with a K_{D} less than 10⁻⁶M, or less than 10⁻⁷M, less than 5x10⁻⁸M, less than 10⁻⁸M or less than 10⁻⁹M. The unaltered reference protein either will not meaningfully bind to VEGFR2 or will bind with a K_{D} of greater than 10⁻⁶M. The non-antibody polypeptide may inhibit VEGFR2 signaling, particularly where the non-antibody polypeptide has a K_{D} of less than 5x10⁻⁸M, less than 10⁻⁸M or less than 10⁻⁹M, although higher K_{D} values may be tolerated where the k_{off} is sufficiently low (e.g., less than 5x10⁻⁴ s⁻¹). The immunoglobulin-like fold may be a ¹⁰Fn3 polypeptide.

In certain embodiments, the disclosure provides a polypeptide comprising a single domain having an immunoglobulin fold that binds to VEGFR2. The polypeptide may have a molecular weight of less than 20 kDa, or less than 15 kDa and will generally be derived (by, for example, alteration of the amino acid sequence) from a variable domain of an immunoglobulin. The polypeptide may bind VEGFR2 with a K_{D} less than 10⁻⁶M, or less than 10⁻⁷M, less than 5x10⁻⁸M, less than 10⁻⁸M or less than 10⁻⁹M. The polypeptide may inhibit VEGFR2 signaling, particularly where the polypeptide has a K_{D} of less than 5x10⁻⁸M, less than 10⁻⁸M or less than 10^{- 9}M, although higher K_{D} values may be tolerated where the k_{off} is sufficiently low or where the kₒₙ is sufficiently high. In certain preferred embodiments, a single domain polypeptide having an immunoglobulin fold derived from an immunoglobulin light chain variable domain and capable of binding to VEGFR2 may comprise an amino acid sequence selected from the VEGFR2 binding clones described herein.

A PEG moiety or other moiety of interest may be covalently bound to the cysteine at position from about 85 to 100 depending on the protein. The PEG moiety may also be covalently bonded to an amine moiety in the polypeptide. The amine moiety may be, for example, a primary amine found at the N-terminus of a polypeptide or an amine group present in an amino acid, such as lysine or arginine. In certain embodiments, the PEG moiety is attached at a position on the polypeptide selected from the group consisting of: a) the N-terminus; b) between the N-terminus and the most N-terminal beta strand or beta-like strand; c) a loop positioned on a face of the polypeptide opposite the target-binding site; d) between the C-terminus and the most C-terminal beta strand or beta-like strand; and e) at the C-terminus.

In certain embodiments, any of the VEGFR2 binding polypeptides described herein may be bound to one or more additional moieties, including, for example, a moiety that also binds to VEGFR2 (e.g., a second identical or different VEGFR2 binding polypeptide), a moiety that binds to a different target (e.g., to create a dual-specificity binding agent), a labeling moiety, a moiety that facilitates protein purification or a moiety that provides improved pharmacokinetics. Improved pharmacokinetics may be assessed according to the perceived therapeutic need. Often it is desirable to increase bioavailability and/or increase the time between doses, possibly by increasing the time that a protein remains available in the serum after dosing. In some instances, it is desirable to improve the continuity of the serum concentration of the protein over time (e.g., decrease the difference in serum concentration of the protein shortly after administration and shortly before the next administration). Moieties that tend to slow clearance of a protein from the blood, herein referred to as "PK moieties", include polyoxyalkylene moieties, e.g., polyethylene glycol, sugars (e.g., sialic acid), and well-tolerated protein moieties (e.g., Fc, Fc fragments or serum albumin). The polypeptides of the invention may be fused to albumin or a fragment (portion) or variant of albumin as described in U.S. Publication No. 20070048282.The single domain polypeptide may be attached to a moiety that reduces the clearance rate of the polypeptide in a mammal (e.g., mouse, rat, or human) by greater than three-fold relative to the unmodified polypeptide. Other measures of improved pharmacokinetics may include serum half-life, which is often divided into an alpha phase and a beta phase. Either or both phases may be improved significantly by addition of an appropriate moiety. Where polyethylene glycol is employed, one or more PEG molecules may be attached at different positions in the protein, and such attachment may be achieved by reaction with amines, thiols or other suitable reactive groups. Pegylation may be achieved by site-directed pegylation, wherein a suitable reactive group is introduced into the protein to create a site where pegylation preferentially occurs. In a preferred embodiment, the protein is modified so as to have a cysteine residue at a desired position, permitting site directed pegylation on the cysteine. PEG may vary widely in molecular weight and may be branched or linear. Notably, the present disclosure establishes that pegylation is compatible with target binding activity of ¹⁰Fn3 polypeptides and, further, that pegylation improves the pharmacokinetics of such polypeptides. Accordingly, in one embodiment, the disclosure provides pegylated forms of ¹⁰Fn3 polypeptides, regardless of the target that can be bound by such polypeptides.

In some embodiments, the polypeptide of the invention comprises a conjugate of a fibronectin-based scaffold protein and a PK moiety. The fibronectin-based scaffold protein may bind any human protein and is preferably derived from a ¹⁰Fn3 domain. The PK moiety may be any moiety that improves the pharmacokinetics of the fibronectin-based scaffold protein. In some embodiments, the PK moiety at least doubles the serum half-life of the scaffold protein. In some embodiments, the PK moiety is linked to the scaffold protein via a polypeptide linker. Exemplary polypeptide linkers include PSTSTST (SEQ ID NO: 63), EIDKPSQ (SEQ ID NO: 64), and GS linkers, such as GSGSGSGSGS (SEQ ID NO: 65) and multimers thereof. In some embodiments the PK moiety is human serum albumin. In some embodiments, the PK moiety is transferrin.

In certain aspects, the disclosure provides methods for using an VEGFR2 binding protein to inhibit VEGFR2 biological activity in a cell or to inhibit a biological activity mediated by VEGFR2. The cell may be situated in vivo or ex vivo, and may be, for example, a cell of a living organism, a cultured cell or a cell in a tissue sample. The method may comprise contacting said cell with any of the VEGFR2-inhibiting polypeptides disclosed herein, in an amount and for a time sufficient to inhibit such biological activity.

In certain aspects, the disclosure provides methods for treating a subject having a condition which responds to the inhibition of VEGFR2. Such a method may comprise administering to said subject an effective amount of any of the VEGFR2 inhibiting polypeptides described herein. A condition may be one that is characterized by inappropriate VEGFR2 biology. Any of the VEGFR2 inhibiting polypeptides described herein may be used for the preparation of a medicament for the treatment of a disorder, particularly a disorder selected from the group consisting of: an autoimmune disorder, a restenosis, and a cancer.

In certain aspects, the disclosure provides methods for detecting VEGFR2 in a sample. A method may comprise contacting the sample with a VEGFR2 binding polypeptide described herein, wherein said contacting is carried out under conditions that allow polypeptide-VEGFR2 complex formation; and detecting said complex, thereby detecting said VEGFR2 in said sample. Detection may be carried out using any technique known in the art, such as, for example, radiography, immunological assay, fluorescence detection, mass spectroscopy, or surface plasmon resonance. The sample will often by a biological sample, such as a biopsy, and particularly a biopsy of a tumor, a suspected tumor. The sample may be from a human or other mammal. The VEGFR2 binding polypeptide may be labeled with a labeling moiety, such as a radioactive moiety, a fluorescent moiety, a chromogenic moiety, a chemiluminescent moiety, or a hapten moiety. The VEGFR2 binding polypeptide may be immobilized on a solid support.

Another aspect of the disclosure relates to a nucleic acid comprising a nucleic acid sequence encoding a polypeptide disclosed herein. In certain embodiments, a nucleic acid may comprise a nucleic acid sequence encoding a polypeptide selected from the group consisting of any of the protein sequences in the tables disclosed herein.

A further aspect of the disclosure relates to an expression vector comprising a nucleic acid operably linked with a promoter, wherein the nucleic acid encodes a polypeptide disclosed herein. Another aspect of the disclosure relates to a cell comprising a nucleic acid disclosed herein. Also provided is a method of producing the polypeptide that binds VEGFR2 comprising: expressing a nucleic acid encoding a polypeptide of the disclosure. In certain embodiments, the nucleic acid may comprise a sequence that encodes a polypeptide selected from the group consisting of any of the sequences in the tables disclosed herein and their corresponding proteins. In certain embodiments, the nucleic acid is expressed in a cell. Alternatively, the nucleic acid is expressed in a cell-free system.

In certain aspects, the disclosure provides discoveries that may be applicable to any ¹⁰Fn3 polypeptide, regardless of which target the polypeptide is engineered to bind. As noted above, the disclosure demonstrates that PEG can be used successfully to improve the pharmacokinetics of a ¹⁰Fn3 polypeptide, while not interfering meaningfully with target binding. Accordingly, the disclosure provides pegylated ¹⁰Fn3 polypeptides that bind to target and have improved pharmacokinetics relative to the non-pegylated polypeptide. In a further embodiment, the disclosure demonstrates that a deletion of the first eight amino acids of a ¹⁰Fn3 polypeptide can increase target binding affinity. Accordingly, the disclosure provides ¹⁰Fn3 polypeptides lacking the initial eight amino acids (amino acids numbered in reference to the sequence of SEQ ID NO:1). It is understood that one or two amino acids may be added back to the deleted form of the polypeptide so as to facilitate translation and proper processing. The disclosure demonstrates that subcutaneous administration of a ¹⁰Fn3 polypeptide results in a delayed release of polypeptide into the bloodstream and a decreased maximum serum concentration of the ¹⁰Fn3 polypeptide. Accordingly, the disclosure provides methods for administering a ¹⁰Fn3 polypeptide to a patient by a subcutaneous administration. This route of administration may be useful to achieve a delayed release relative to intravenous administration, and/or to decrease the maximum serum concentration of the ¹⁰Fn3 polypeptide by at least 25% or at least 50% relative to the maximum serum concentration achieved by intravenous administration of an equal dosage. The administered ¹⁰Fn3 polypeptide may be attached to a moiety that increases the serum half-life (or decreases clearance rate, or similarly affects another pharmacokinetic parameter) of the ¹⁰Fn3 polypeptide, such as a polyethylene glycol moiety.

In certain aspects, the disclosure provides single domain polypeptides that bind to a preselected target protein from a first mammal and to a homolog thereof from a second mammal. Such single domain polypeptides are particularly useful where the first mammal is a human and the second mammal is a desirable mammal in which to conduct preclinical testing, such as a mouse, rat, guinea pig, dog, or non-human primate. The disclosure demonstrates that single domain polypeptides can be engineered to have such dual specificity, and that the dual specificity simplifies drug development by allowing testing of the same polypeptide in human cells, human subjects and animal models. Preferably, the preselected target protein of the first mammal and the homolog thereof from the second mammal are sufficiently similar in amino acid sequence to allow generation of dual specificity polypeptides. For example, the preselected target protein and the homolog from the second mammal may share at least 80%, 90%, or 95% identity across a region of at least 50 amino acids, and optionally may share at least 80%, 90%, or 95% identity across the entire protein sequence or across the sequence of the extracellular domain, in the case of a membrane protein. A single domain polypeptide with this type of dual specificity binding characteristic may comprise an immunoglobulin or immunoglobulin-like domain, and will preferably bind to both the preselected human target protein and to the homolog thereof with a dissociation constant of less than 1x10⁻⁶M, 1x10⁻⁷M, 5x10⁻⁸M, 1x10⁻⁸M or 1x10⁻⁹M.

### Additional Bispecific and Multi-Specific Embodiments

In many embodiments it will be desirable to make multi-specific compositions, e.g. compositions that bind more than one target or other protein of interest. In one aspect, proteins of the invention comprise a first protein with a binding affinity of about 10 nM (other appropriate affinity described herein) to first desired target (e.g. VEGFR-2) or less and binds an undesired, related target (e.g. VEGFR-1 and VEGFR-3) with a binding affinity of about 1 µM (other appropriate affinity described herein) or greater and is preferably a single domain or substantially monovalent and is linked to or attached to a second protein with a binding affinity of about 10 nM (other appropriate affinity described herein) to a second desired target (e.g. EGFR, c-Met, c-kit, Her2, FGFR1, VEGF-A, VEGF-C, VEGF-D, folate receptor) or less and binds an undesired, related target (e.g. human insulin receptor) with a binding affinity of about 1 µM (other appropriate affinity described herein) or greater and is preferably a single domain or substantially monovalent. Such molecules with bispecific affinity can be further attached to other molecules, including other proteins described herein.

In one aspect, proteins of the invention comprise a first protein with a binding affinity of about 10 nM (other appropriate affinity described herein) to first desired target (e.g. VEGFR-2) or less and binds an undesired, related target (e.g. VEGFR-1 and VEGFR-3) with a binding affinity of about 1 µM (other appropriate affinity described herein) or greater and is preferably a single domain or substantially monovalent and is linked to a second protein with a binding affinity of about 10 nM (other appropriate affinity described herein) to a second desired target (e.g. VEGFR-1) or less and binds an undesired, related target (e.g. VEGFR-2 and VEGFR-3) with a binding affinity of about 1 µM (other appropriate affinity described herein) or greater and is preferably a single domain or substantially monovalent. Such molecules with bispecific affinity can be further attached to other molecules, including other proteins described herein.

### Additional PEG Embodiments

In one aspect of the invention, PEG (or functionally similar molecule) can be used to connect two proteins that are non-antibody moieties that bind a single target, particularly proteins wherein each binding protein is comprised of a single domain or multiple domains, usually wherein each domain is about 50 or about 60 or about 75 amino acids or more (as opposed to small peptides of 5 to 20 amino acids). Preferably fibronectin based scaffolds, such as Adnectins^{™}, can be used advantageously in such embodiments and more preferably with the proper engineering of Cys or Lys amino acids.

In addition, nonPEG and PEG aspects of the invention include antibody moieties (e.g., camel antibodies and their derivatives, as well as single chain and domain antibodies; and particularly those expressed from microbes) and antibody-like moieties (e.g., derivatives of lipocalins, ankyrins, multiple Cys-Cys domains, and tetranectins; and particularly those expressed from microbes), particularly those less than about 40 kDa that are connect by PEG, and more particularly those that have a limited number of cys amino acids.

There are many properties and advantages of PEG linked proteins of the invention not previously recognized or discovered. When such proteins are expressed in microbes it may be preferable to isolate domains and then link them via PEG or other polymeric linker. PEG, or other functionally operably polymeric linkers, can be used to optimally vary the distance between each protein moiety to create a protein with one or more of the following characteristics: 1) reduced or increased steric hindrance of binding of one or more protein domain when binding to a protein of interest (e.g., a target), 2) connect two or more domains that bind different targets, 3) increase protein stability or solubility without searching for additional amino acid substitutions to increase stability or solubility (e.g., solubility at least about 20mg/ml, or at least about 50mg/ml), 4) decrease protein aggregation without searching for additional amino acid substitutions to decrease stability (e.g., as measured by SEC), 4) increase the overall avidity or affinity of the protein for the protein of interest by adding additional binding domains. Additional advantages of PEG linked proteins include rapidly making monospecific, multi-valent binding modes, as well as multi-specific, monovalent or multivalent binding modes depending on the number of protein targeting moieties that are included in the PEG linked protein.

¹⁰Fn3 polypeptides of the invention can be pegylated and retain ligand binding activity. In a preferred embodiment, the pegylated ¹⁰Fn3 polypeptide is produced by site-directed pegylation, particularly by conjugation of PEG to a cysteine moiety at the N- or C-terminus. Accordingly, the present disclosure provides a target-binding ¹⁰Fn3 polypeptide with improved pharmacokinetic properties, the polypeptide comprising: a ¹⁰Fn3 domain having from about 80 to about 150 amino acids, wherein at least one of the loops of said ¹⁰Fn3 domain participate in target binding; and a covalently bound PEG moiety, wherein said ¹⁰Fn3 polypeptide binds to the target with a K_{D} of less than 100 nM and has a clearance rate of less than 30 mL/hr/kg in a mammal. The PEG moiety may be attached to the ¹⁰Fn3 polypeptide by site directed pegylation, such as by attachment to a Cys residue, where the Cys residue may be positioned at the N-terminus of the ¹⁰Fn3 polypeptide or between the N-terminus and the most N-terminal beta or beta-like strand or at the C-terminus of the ¹⁰Fn3 polypeptide or between the C-terminus and the most C-terminal beta or beta-like strand. A Cys residue may be situated at other positions as well, particularly any of the loops that do not participate in target binding. A PEG moiety may also be attached by other chemistry, including by conjugation to amines. In addition, the invention includes this type of N or C terminal PEG conjugation to antibody moieties (e.g., camel antibodies and their derivatives, as well as single chain and domain antibodies; and particularly those expressed from microbes) and antibody-like moieties (e.g., derivatives of lipocalins, ankyrins, multiple Cys-Cys domains, and tetranectins; and particularly those expressed from microbes), particularly those less than 40 kDa that are connect by PEG, and more particularly those that have a limited number of cys amino acids.

In one specific embodiment of the present invention, modified forms of the subject soluble polypeptides comprise linking the subject soluble polypeptides to nonproteinaceous polymers. In one specific embodiment, the polymer is polyethylene glycol ("PEG"), polypropylene glycol, or polyoxyalkylenes, in the manner as set forth in U.S. Patent Nos. 4,640,835; 4,496,689; 4,301,144; 4,670,417; 4,791,192 or 4,179,337. Examples of the modified polypeptide of the invention include PEGylated proteins further described herein.

PEG is a well-known, water soluble polymer that is commercially available or can be prepared by ring-opening polymerization of ethylene glycol according to methods well known in the art (Sandler and Karo, Polymer Synthesis, Academic Press, New York, Vol. 3, pages 138-161). The term "PEG" is used broadly to encompass any polyethylene glycol molecule, without regard to size or to modification at an end of the PEG, and can be represented by the formula: X-O(CH₂CH₂O)ₙ₋₁CH₂CH₂OH (1), where n is 20 to 2300 and X is H or a terminal modification, e.g., a C₁₋₄ alkyl. In one embodiment, the PEG of the invention terminates on one end with hydroxy or methoxy, i.e., X is H or CH₃ ("methoxy PEG"). A PEG can contain further chemical groups which are necessary for binding reactions; which results from the chemical synthesis of the molecule; or which is a spacer for optimal distance of parts of the molecule. In addition, such a PEG can consist of one or more PEG side-chains which are linked together. PEGs with more than one PEG chain are called multiarmed or branched PEGs. Branched PEGs can be prepared, for example, by the addition of polyethylene oxide to various polyols, including glycerol, pentaerythriol, and sorbitol. For example, a four-armed branched PEG can be prepared from pentaerythriol and ethylene oxide. Branched PEG are described in, for example, European Published Application No. 473084A and U.S. Patent No. 5,932,462. One form of PEGs includes two PEG side-chains (PEG2) linked via the primary amino groups of a lysine (Monfardini, C., et al., Bioconjugate Chem. 6 (1995) 62-69).

In a preferred embodiment, the pegylated ¹⁰Fn3 polypeptide is produced by site-directed pegylation, particularly by conjugation of PEG to a cysteine moiety at the N- or C-terminus. Accordingly, the present disclosure provides a target-binding ¹⁰Fn3 polypeptide with improved pharmacokinetic properties, the polypeptide comprising: a ¹⁰Fn3 domain having from about 80 to about 150 amino acids, wherein at least one of the loops of said ¹⁰Fn3 domain participate in target binding; and a covalently bound PEG moiety, wherein said ¹⁰Fn3 polypeptide binds to the target with a K_{D} of less than 100 nM and has a clearance rate of less than 30 mL/hr/kg in a mammal. The PEG moiety may be attached to the ¹⁰Fn3 polypeptide by site directed pegylation, such as by attachment to a Cys residue, where the Cys residue may be positioned at the N-terminus of the ¹⁰Fn3 polypeptide or between the N-terminus and the most N-terminal beta or beta-like strand or at the C-terminus of the ¹⁰Fn3 polypeptide or between the C-terminus and the most C-terminal beta or beta-like strand. A Cys residue may be situated at other positions as well, particularly any of the loops that do not participate in target binding. A PEG moiety may also be attached by other chemistry, including by conjugation to amines.

PEG conjugation to peptides or proteins generally involves the activation of PEG and coupling of the activated PEG-intermediates directly to target proteins/peptides or to a linker, which is subsequently activated and coupled to target proteins/peptides (see Abuchowski, A. et al, J. Biol. Chem., 252, 3571 (1977) and J. Biol. Chem., 252, 3582 (1977), Zalipsky, et al., and Harris et. al., in: Poly(ethylene glycol) Chemistry: Biotechnical and Biomedical Applications; (J. M. Harris ed.) Plenum Press: New York, 1992; Chap.21 and 22). It is noted that a binding polypeptide containing a PEG molecule is also known as a conjugated protein, whereas the protein lacking an attached PEG molecule can be referred to as unconjugated.

A variety of molecular mass forms of PEG can be selected, e.g., from about 1,000 Daltons (Da) to 100,000 Da (n is 20 to 2300), for conjugating to binding polypeptides of the invention. The number of repeating units "n" in the PEG is approximated for the molecular mass described in Daltons. It is preferred that the combined molecular mass of PEG on an activated linker is suitable for pharmaceutical use. Thus, in one embodiment, the molecular mass of the PEG molecules does not exceed 100,000 Da. For example, if three PEG molecules are attached to a linker, where each PEG molecule has the same molecular mass of 12,000 Da (each n is about 270), then the total molecular mass of PEG on the linker is about 36,000 Da (total n is about 820). The molecular masses of the PEG attached to the linker can also be different, e.g., of three molecules on a linker two PEG molecules can be 5,000 Da each (each n is about 110) and one PEG molecule can be 12,000 Da (n is about 270).

In a specific embodiment of the invention, a VEGFR2 binding polypeptide is covalently linked to one poly(ethylene glycol) group of the formula: -CO- (CH₂)ₓ- (OCH₂CH₂)ₘ-OR , with the -CO (i.e. carbonyl) of the poly(ethylene glycol) group forming an amide bond with one of the amino groups of the binding polypeptide; R being lower alkyl; x being 2 or 3; m being from about 450 to about 950; and n and m being chosen so that the molecular weight of the conjugate minus the binding polypeptide is from about 10 to 40 kDa. In one embodiment, a binding polypeptide's ε-amino group of a lysine is the available (free) amino group.

The above conjugates may be more specifically presented by formula (II): P-NHCO-(CH₂)ₓ-(OCH₂CH₂)ₘ-OR (II), wherein P is the group of a binding polypeptide as described herein, (i.e. without the amino group or amino groups which form an amide linkage with the carbonyl shown in formula (II); and wherein R is lower alkyl; x is 2 or 3; m is from about 450 to about 950 and is chosen so that the molecular weight of the conjugate minus the binding polypeptide is from about 10 to about 40 kDa. As used herein, the given ranges of "m" have an orientational meaning. The ranges of "m" are determined in any case, and exactly, by the molecular weight of the PEG group.

One skilled in the art can select a suitable molecular mass for PEG, e.g., based on how the pegylated binding polypeptide will be used therapeutically, the desired dosage, circulation time, resistance to proteolysis, immunogenicity, and other considerations. For a discussion of PEG and its use to enhance the properties of proteins, see N. V. Katre, Advanced Drug Delivery Reviews 10: 91-114 (1993).

In one embodiment of the invention, PEG molecules may be activated to react with amino groups on a binding polypeptide, such as with lysines (Bencham C. O. et al., Anal. Biochem., 131, 25 (1983); Veronese, F. M. et al., Appl. Biochem., 11, 141 (1985).; Zalipsky, S. et al., Polymeric Drugs and Drug Delivery Systems, adrs 9-110 ACS Symposium Series 469 (1999); Zalipsky, S. et al., Europ. Polym. J., 19, 1177-1183 (1983); Delgado, C. et al., Biotechnology and Applied Biochemistry, 12, 119-128 (1990)).

In one specific embodiment, carbonate esters of PEG are used to form the PEG-binding polypeptide conjugates. N,N'-disuccinimidylcarbonate (DSC) may be used in the reaction with PEG to form active mixed PEG-succinimidyl carbonate that may be subsequently reacted with a nucleophilic group of a linker or an amino group of a binding polypeptide (see U.S. Patent No. 5,281,698 and U.S. Patent No. 5,932,462). In a similar type of reaction, 1,1'-(dibenzotriazolyl)carbonate and di-(2-pyridyl)carbonate may be reacted with PEG to form PEG-benzotriazolyl and PEG-pyridyl mixed carbonate (U.S. Patent No. 5,382,657), respectively.

Pegylation of a ¹⁰Fn3 polypeptide can be performed according to the methods of the state of the art, for example by reaction of the binding polypeptide with electrophilically active PEGs (supplier: Shearwater Corp., USA, www.shearwatercorp.com). Preferred PEG reagents of the present invention are, e.g., N-hydroxysuccinimidyl propionates (PEG-SPA), butanoates (PEG-SBA), PEG-succinimidyl propionate or branched N-hydroxysuccinimides such as mPEG2-NHS (Monfardini, C., et al., Bioconjugate Chem. 6 (1995) 62-69). Such methods may used to pegylated at an ε-amino group of a binding polypeptide lysine or the N-terminal amino group of the binding polypeptide.

In another embodiment, PEG molecules may be coupled to sulfhydryl groups on a binding polypeptide (Sartore, L., et al., Appl. Biochem. Biotechnol., 27, 45 (1991); Morpurgo et al., Biocon. Chem., 7, 363-368 (1996); Goodson et al., Bio/Technology (1990) 8, 343; U.S. Patent No. 5,766,897). U.S. Patent Nos. 6,610,281 and 5,766,897 describes exemplary reactive PEG species that may be coupled to sulfhydryl groups.

In some embodiments where PEG molecules are conjugated to cysteine residues on a binding polypeptide, the cysteine residues are native to the binding polypeptide, whereas in other embodiments, one or more cysteine residues are engineered into the binding polypeptide. Mutations may be introduced into an binding polypeptide coding sequence to generate cysteine residues. This might be achieved, for example, by mutating one or more amino acid residues to cysteine. Preferred amino acids for mutating to a cysteine residue include serine, threonine, alanine and other hydrophilic residues. Preferably, the residue to be mutated to cysteine is a surface-exposed residue. Algorithms are well-known in the art for predicting surface accessibility of residues based on primary sequence or a protein. Alternatively, surface residues may be predicted by comparing the amino acid sequences of binding polypeptides, given that the crystal structure of the framework based on which binding polypeptides are designed and evolved has been solved (see Himanen et al., Nature. (2001) 20-27;414(6866):933-8) and thus the surface-exposed residues identified. In one embodiment, cysteine residues are introduced into binding polypeptides at or near the N- and/or C-terminus, or within loop regions.

In some embodiments, the pegylated binding polypeptide comprises a PEG molecule covalently attached to the alpha amino group of the N-terminal amino acid. Site specific N-terminal reductive amination is described in Pepinsky et al., (2001) JPET, 297,1059, and U.S. Patent No. 5,824,784. The use of a PEG-aldehyde for the reductive amination of a protein utilizing other available nucleophilic amino groups is described in U.S. Patent No. 4,002,531, in Wieder et al., (1979) J. Biol. Chem. 254,12579, and in Chamow et al., (1994) Bioconjugate Chem. 5, 133.

In another embodiment, pegylated binding polypeptide comprises one or more PEG molecules covalently attached to a linker, which in turn is attached to the alpha amino group of the amino acid residue at the N-terminus of the binding polypeptide. Such an approach is disclosed in U.S. Publication No. 2002/0044921 and PCT Publication No. WO94/01451.

In one embodiment, a binding polypeptide is pegylated at the C-terminus. In a specific embodiment, a protein is pegylated at the C-terminus by the introduction of C-terminal azido-methionine and the subsequent conjugation of a methyl-PEG-triarylphosphine compound via the Staudinger reaction. This C-terminal conjugation method is described in Cazalis et al., C-Terminal Site-Specific PEGylation of a Truncated Thrombomodulin Mutant with Retention of Full Bioactivity, Bioconjug Chem. 2004;15(5):1005-1009.

Monopegylation of a binding polypeptide can also be produced according to the general methods described in PCT Publication No. WO94/01451 WO94/01451 describes a method for preparing a recombinant polypeptide with a modified terminal amino acid alpha-carbon reactive group. The steps of the method involve forming the recombinant polypeptide and protecting it with one or more biologically added protecting groups at the N-terminal alpha-amine and C-terminal alpha-carboxyl. The polypeptide can then be reacted with chemical protecting agents to selectively protect reactive side chain groups and thereby prevent side chain groups from being modified. The polypeptide is then cleaved with a cleavage reagent specific for the biological protecting group to form an unprotected terminal amino acid alpha-carbon reactive group. The unprotected terminal amino acid alpha-carbon reactive group is modified with a chemical modifying agent. The side chain protected terminally modified single copy polypeptide is then deprotected at the side chain groups to form a terminally modified recombinant single copy polypeptide. The number and sequence of steps in the method can be varied to achieve selective modification at the N- and/or C-terminal amino acid of the polypeptide.

The ratio of a binding polypeptide to activated PEG in the conjugation reaction can be from about 1:0.5 to 1:50, between from about 1:1 to 1:30, or from about 1:5 to 1:15. Various aqueous buffers can be used in the present method to catalyze the covalent addition of PEG to the binding polypeptide. In one embodiment, the pH of a buffer used is from about 7.0 to 9.0. In another embodiment, the pH is in a slightly basic range, *e.g.,* from about 7.5 to 8.5. Buffers having a pKa close to neutral pH range may be used, e.g., phosphate buffer. Other ratios will be used when making multi-specific PEG linked proteins, such as about 1:4 to 1:8, or about 1:3 to 1:5

Conventional separation and purification techniques known in the art can be used to purify PEGylated binding polypeptide, such as size exclusion (e.g., gel filtration) and ion exchange chromatography. Products may also be separated using SDS-PAGE. Products that may be separated include mono-, di-, tri- poly- and un- pegylated binding polypeptide, as well as free PEG. The percentage of mono-PEG conjugates can be controlled by pooling broader fractions around the elution peak to increase the percentage of mono-PEG in the composition. About ninety percent mono-PEG conjugates represents a good balance of yield and activity. Compositions in which, for example, at least ninety-two percent or at least ninety-six percent of the conjugates are mono-PEG species may be desired. In an embodiment of this invention the percentage of mono-PEG conjugates is from ninety percent to ninety-six percent.

In one embodiment, PEGylated binding polypeptide of the invention contain one, two or more PEG moieties. In one embodiment, the PEG moiety(ies) are bound to an amino acid residue which is on the surface of the protein and/or away from the surface that contacts the target ligand. In one embodiment, the combined or total molecular mass of PEG in PEG- binding polypeptide is from about 3,000 Da to 60,000 Da, optionally from about 10,000 Da to 36,000 Da. In a one embodiment, the PEG in pegylated binding polypeptide is a substantially linear, straight-chain PEG.

In one embodiment of the invention, the PEG in pegylated binding polypeptide is not hydrolyzed from the pegylated amino acid residue using a hydroxylamine assay, e.g., 450 mM hydroxylamine (pH 6.5) over 8 to 16 hours at room temperature, and is thus stable. In one embodiment, greater than 80% of the composition is stable mono-PEG-binding polypeptide, more preferably at least 90%, and most preferably at least 95%.

In another embodiment, the pegylated binding polypeptides of the invention will preferably retain at least about 25%, 50%, 60%, 70%, 80%, 85%, 90%, 95% or 100% of the biological activity associated with the unmodified protein. In one embodiment, biological activity refers to its ability to bind to VEGFR-2, as assessed by K_{D}, kₒₙ or k_{off}. In one specific embodiment, the pegylated binding polypeptide protein shows an increase in binding to VEGFR2 relative to unpegylated binding polypeptide.

The serum clearance rate of PEG-modified polypeptide may be decreased by about 10%, 20%, 30%, 40%, 50%, 60%, 70%, 80%, or even 90%, relative to the clearance rate of the unmodified binding polypeptide. The PEG-modified polypeptide may have a half-life (t_{1/2}) which is enhanced relative to the half-life of the unmodified protein. The half-life of PEG-binding polypeptide may be enhanced by at least 10%, 20%, 30%, 40%, 50%, 60%, 70%, 80%, 90%, 100%, 125%, 150%, 175%, 200%, 250%, 300%, 400% or 500%, or even by 1000% relative to the half-life of the unmodified binding polypeptide. In some embodiments, the protein half-life is determined in vitro, such as in a buffered saline solution or in serum. In other embodiments, the protein half-life is an *in vivo* half life, such as the half-life of the protein in the serum or other bodily fluid of an animal.

### Additional Vectors & Polynucleotides Embodiments

Nucleic acids encoding any of the various proteins or polypeptides disclosed herein may be synthesized chemically. Codon usage may be selected so as to improve expression in a cell. Such codon usage will depend on the cell type selected. Specialized codon usage patterns have been developed for E. coli and other bacteria, as well as mammalian cells, plant cells, yeast cells and insect cells. See for example: Mayfield et al., Proc Natl Acad Sci U S A. 2003 Jan 21;100(2):438-42; Sinclair et al. Protein Expr Purif. 2002 Oct;26(1):96-105; Connell ND. Curr Opin Biotechnol. 2001 Oct;12(5):446-9; Makrides et al. Microbiol Rev. 1996 Sep;60(3):512-38; and Sharp et al. Yeast. 1991 Oct;7(7):657-78.

General techniques for nucleic acid manipulation are described for example in Sambrook et al., Molecular Cloning: A Laboratory Manual, Vols. 1-3, Cold Spring Harbor Laboratory Press, 2 ed., 1989, or F. Ausubel et al., Current Protocols in Molecular Biology (Green Publishing and Wiley-Interscience: New York, 1987) and periodic updates. The DNA encoding the polypeptide is operably linked to suitable transcriptional or translational regulatory elements derived from mammalian, viral, or insect genes. Such regulatory elements include a transcriptional promoter, an optional operator sequence to control transcription, a sequence encoding suitable mRNA ribosomal binding sites, and sequences that control the termination of transcription and translation. The ability to replicate in a host, usually conferred by an origin of replication, and a selection gene to facilitate recognition of transformants are additionally incorporated.

The proteins of this invention may be produced recombinantly not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed (i.e., cleaved by a signal peptidase) by the host cell. For prokaryotic host cells that do not recognize and process a native signal sequence, the signal sequence is substituted by a prokaryotic signal sequence selected, for example, from the group of the alkaline phosphatase, penicillinase, 1pp, or heat-stable enterotoxin II leaders. For yeast secretion the native signal sequence may be substituted by, e.g., the yeast invertase leader, a factor leader (including Saccharomyces and Kluyveromyces .alpha.-factor leaders), or acid phosphatase leader, the C. albicans glucoamylase leader, or the signal described in PCT Publication No. WO90/13646. In mammalian cell expression, mammalian signal sequences as well as viral secretory leaders, for example, the herpes simplex gD signal, are available. The DNA for such precursor regions may be ligated in reading frame to DNA encoding the protein.

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2.mu. plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for Bacilli.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian or eukaryotic cells are those that enable the identification of cells competent to take up the multivalent antibody nucleic acid, such as DHFR, thymidine kinase, metallothionein-I and -II, preferably primate metallothionein genes, adenosine deaminase, ornithine decarboxylase, etc.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity.

Alternatively, host cells (particularly wild-type hosts that contain endogenous DHFR) transformed or co-transformed with DNA sequences encoding multivalent antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Patent No. 4,965,199.

A suitable selection gene for use in yeast is the trp1 gene present in the yeast plasmid YRp7 (Stinchcomb et al., Nature, 282:39 (1979)). The trp1 gene provides a selection marker for a mutant strain of yeast lacking the ability to grow in tryptophan, for example, ATCC No. 44076 or PEP4-1. Jones, Genetics, 85:12 (1977). The presence of the trp1 lesion in the yeast host cell genome then provides an effective environment for detecting transformation by growth in the absence of tryptophan. Similarly, Leu2-deficient yeast strains (ATCC 20,622 or 38,626) are complemented by known plasmids bearing the Leu2 gene.

In addition, vectors derived from the 1.6 .mu.m circular plasmid pKD1 can be used for transformation of Kluyveromyces yeasts. Alternatively, an expression system for large-scale production of recombinant calf chymosin was reported for K. lactis. Van den Berg, Bio/Technology, 8:135 (1990). Stable multi-copy expression vectors for secretion of mature recombinant human serum albumin by industrial strains of Kluyveromyces have also been disclosed. Fleer et al., Bio/Technology, 9:968-975 (1991).

Expression and cloning vectors usually contain a promoter that is recognized by the host organism and is operably linked to the nucleic acid encoding the protein of the invention, e.g., a fibronectin-based scaffold protein. Promoters suitable for use with prokaryotic hosts include the phoA promoter, beta-lactamase and lactose promoter systems, alkaline phosphatase, a tryptophan (trp) promoter system, and hybrid promoters such as the tac promoter. However, other known bacterial promoters are suitable. Promoters for use in bacterial systems also will contain a Shine-Dalgarno (S.D.) sequence operably linked to the DNA encoding the protein of the invention.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP Patent Publication No. 73,657. Yeast enhancers also are advantageously used with yeast promoters.

Transcription from vectors in mammalian host cells can be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, e.g., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in . U.S. Patent No. 4,601,978. See also Reyes et al., Nature 297:598-601 (1982) on expression of human .beta.-interferon cDNA in mouse cells under the control of a thymidine kinase promoter from herpes simplex virus. Alternatively, the rous sarcoma virus long terminal repeat can be used as the promoter.

Transcription of a DNA encoding proteins of the invention by higher eukaryotes is often increased by inserting an enhancer sequence into the vector. Many enhancer sequences are now known from mammalian genes (globin, elastase, albumin, .alpha.-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the multivalent antibody-encoding sequence, but is preferably located at a site 5' from the promoter.

Expression vectors used in eukaryotic host cells (e.g., yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding the multivalent antibody. One useful transcription termination component is the bovine growth hormone polyadenylation region. See WO94/11026 and the expression vector disclosed therein.

The recombinant DNA can also include any type of protein tag sequence that may be useful for purifying the protein. Examples of protein tags include but are not limited to a histidine tag, a FLAG tag, a myc tag, an HA tag, or a GST tag. Appropriate cloning and expression vectors for use with bacterial, fungal, yeast, and mammalian cellular hosts can be found in Cloning Vectors: A Laboratory Manual, (Elsevier, New York, 1985).

The expression construct is introduced into the host cell using a method appropriate to the host cell, as will be apparent to one of skill in the art. A variety of methods for introducing nucleic acids into host cells are known in the art, including, but not limited to, electroporation; transfection employing calcium chloride, rubidium chloride, calcium phosphate, DEAE-dextran, or other substances; microprojectile bombardment; lipofection; and infection (where the vector is an infectious agent).

Suitable host cells include prokaryotes, yeast, mammalian cells, or bacterial cells. Suitable bacteria include gram negative or gram positive organisms, for example, E. coli or Bacillus spp. Yeast, preferably from the Saccharomyces species, such as S. cerevisiae, may also be used for production of polypeptides. Various mammalian or insect cell culture systems can also be employed to express recombinant proteins. Baculovirus systems for production of heterologous proteins in insect cells are reviewed by Luckow and Summers, (Bio/Technology, 6:47, 1988). Examples of suitable mammalian host cell lines include endothelial cells, COS-7 monkey kidney cells, CV-1, L cells, C127, 3T3, Chinese hamster ovary (CHO), human embryonic kidney cells, HeLa, 293, 293T, and BHK cell lines. Purified polypeptides are prepared by culturing suitable host/vector systems to express the recombinant proteins. For many applications, the small size of many of the polypeptides disclosed herein would make expression in E. coli as the preferred method for expression. The protein is then purified from culture media or cell extracts.

### Additional Expression & Cell Embodiments

Preferred proteins for production and cell embodiments are fibronectin based scaffolds and related proteins. Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, e.g., E. coli, Enterobacter, Erwinia, Klebsiella, Proteus, Salmonella, e.g., Salmonella typhimurium, Serratia, e.g., Serratia marcescans, and Shigella, as well as Bacilli such as B. subtilis and B. licheniformis (e.g., B. licheniformis 41 P disclosed in DD 266,710 published 12 Apr. 1989), Pseudomonas such as P. aeruginosa, and Streptomyces. One preferred E. coli cloning host is E. coli 294 (ATCC 31,446), although other strains such as E. coli B, E. coli X1776 (ATCC 31,537), and E. coli W3110 (ATCC 27,325) are suitable. These examples are illustrative rather than limiting.

In addition to prokaryotes, eukaryotic microbes such as filamentous fungi or yeast are suitable cloning or expression hosts for protein-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, e.g., K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP Patent Publication No.402,226); Pichia pastoris (EP Patent Publication No. 183,070); Candida; Trichoderma reesia (EP Patent Publication No. 244,234); Neurospora crassa; Schwanniomyces such as Schwanniomyces occidentalis; and filamentous fungi such as, e.g., Neurospora, Penicillium, Tolypocladium, and Aspergillus hosts such as A. nidulans and A. niger.

Suitable host cells for the expression of glycosylated proteins of the invention are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, e.g., the L-1 variant of Autographa californica NPV and the Bm-5 strain of Bombyx mori NPV, and such viruses may be used as the virus herein according to the present invention, particularly for transfection of Spodoptera frugiperda cells.

In some instance it will be desired to produce proteins in vertebrate cells, such as glycosylation, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59. (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; a human hepatoma line (Hep G2); and myeloma or lymphoma cells (e.g., Y0, J558L, P3 and NS0 cells) (see U.S. Patent No. 5,807,715). Plant cell cultures of cotton, corn, potato, soybean, petunia, tomato, and tobacco can also be utilized as hosts.

Host cells are transformed with the herein-described expression or cloning vectors for protein production and cultured in conventional nutrient media modified as appropriate for inducing promoters, selecting transformants, or amplifying the genes encoding the desired sequences.

### Culturing Cells

The host cells used to produce the proteins of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem.102:255 (1980), U.S. Patent Nos. 4,767,704; 4,657,866; 4,927,762; 4,560,655; or 5,122,469; WO90/03430; WO87/00195; or U.S. Patent No. Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

Proteins disclosed herein can also be produced using cell- translation systems. For such purposes the nucleic acids encoding the polypeptide must be modified to allow in vitro transcription to produce mRNA and to allow cell-free translation of the mRNA in the particular cell-free system being utilized (eukaryotic such as a mammalian or yeast cell-free translation system or prokaryotic such as a bacterial cell-free translation system.

Proteins of the invention can also be produced by chemical synthesis (e.g., by the methods described in Solid Phase Peptide Synthesis, 2nd ed., 1984, The Pierce Chemical Co., Rockford, IL). Modifications to the protein can also be produced by chemical synthesis.

The proteins of the present invention can be purified by isolation/purification methods for proteins generally known in the field of protein chemistry. Non-limiting examples include extraction, recrystallization, salting out (e.g., with ammonium sulfate or sodium sulfate), centrifugation, dialysis, ultrafiltration, adsorption chromatography, ion exchange chromatography, hydrophobic chromatography, normal phase chromatography, reversed-phase chromatography, gel filtration, gel permeation chromatography, affinity chromatography, electrophoresis, countercurrent distribution or any combinations of these. After purification, polypeptides may be exchanged into different buffers and/or concentrated by any of a variety of methods known to the art, including, but not limited to, filtration and dialysis.

The purified polypeptide is preferably at least 85% pure, more preferably at least 95% pure, and most preferably at least 98% pure. Regardless of the exact numerical value of the purity, the polypeptide is sufficiently pure for use as a pharmaceutical product.

### Additional Glycosylation Embodiments

In some embodiments it may be preferable to glycosylate proteins of the invention. Preferably, such proteins are fibronectin based scaffolds. Fibronectin based scaffolds do not normally contain glycosylation sites, however, such glycosylation may be engineered into the protein.

Glycosylation of proteins is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. These can be engineered into the proteins of the invention, in particular fibronectin-based scaffold proteins and their corresponding polynucleotides. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the proteins of the invention are conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Nucleic acid molecules encoding such amino acid sequence variants of the proteins of the invention are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the protein (e.g., fibronectin-based scaffold protein).

It may be desirable to modify proteins of the invention with respect to effector function, e.g., so as to enhance antigen-dependent cell-mediated cytotoxicity (ADCC) and/or complement dependent cytotoxicity (CDC) of the antibody. This may be achieved by introducing an active portion of an Fc region, as well as one or more amino acid modifications in an Fc region of the protein (e.g., fibronectin-based scaffold protein), thereby generating a variant Fc region. The Fc region variant may comprise a human Fc region sequence (e.g., a human IgG1, IgG2, IgG3 or IgG4 Fc region) comprising an amino acid modification (e.g., a substitution) at one or more amino acid positions.

In one embodiment, the variant Fc region may mediate antibody-dependent cell-mediated cytotoxicity (ADCC) in the presence of human effector cells more effectively, or bind an Fc gamma receptor (FcγR) with better affinity, than a native sequence Fc region. Such Fc region variants may comprise an amino acid modification at any one or more of positions 256, 290, 298, 312, 326, 330, 333, 334, 360, 378 or 430 of the Fc region, wherein the numbering of the residues in the Fc region is that of the EU index as in Kabat.

### Toxins and Other Molecules Linked to Proteins of the Invention

The proteins of the invention as disclosed herein, may be linked to a cytotoxic agent. Such embodiments can be prepared by in vitro or in vivo methods as appropriate. In vitro methods, include conjugation chemistry well know in the art including chemistry compatible with proteins, such as chemistry for specific amino acids, such as Cys and Lys. In order to link a cytotoxic agent to protein of the invention, a linking group or reactive group is used. Suitable linking groups are well known in the art and include disulfide groups, thioether groups, acid labile groups, photolabile groups, peptidase labile groups and esterase labile groups. Preferred linking groups are disulfide groups and thioether groups. For example, conjugates can be constructed using a disulfide exchange reaction or by forming a thioether bond between the antibody and the cytotoxic agent. Preferred cytotoxic agents are maytansinoids, taxanes and analogs of CC-1065.

In vivo methods include linking toxic, tagging or labeling proteins to proteins of the invention as fusion proteins. A single polypeptide is produced using an encoding polynucleotide for the desired polypeptide. Toxic proteins can be controlled by expressing in toxin resistant or insensitive cells or with inducible promoters in cells that are sensitive.

Although not limiting, in various embodiments, proteins of the invention may be linked to proteins, such as a bacterial toxin, a plant toxin, ricin, abrin, a ribonuclease (RNase), DNase I, a protease, Staphylococcal enterotoxin-A, pokeweed antiviral protein, gelonin, diphtherin toxin, Pseudomonas exotoxin, Pseudomonas endotoxin, Ranpimase (Rap), Rap (N69Q), an enzyme, or a fluorescent protein.

Maytansinoids and maytansinoid analogs are among the preferred cytotoxic agents. Examples of suitable maytansinoids include maytansinol and maytansinol analogs. Suitable maytansinoids are disclosed in U.S. Patent Nos. 4,424,219; 4,256,746; 4,294,757; 4,307,016; 4,313,946; 4,315,929; 4,331,598; 4,361,650; 4,362,663; 4,364,866; 4,450,254; 4,322,348; 4,371,533; 6,333,410; 5,475,092; 5,585,499; and 5,846,545.

Taxanes are also preferred cytotoxic agents. Taxanes suitable for use in the present invention are disclosed in U.S. Patent Nos. 6,372,738 and 6,340,701.

CC-1065 and its analogs are also preferred cytotoxic drugs for use in the present invention. CC-1065 and its analogs are disclosed in U.S. Patent Nos. 6,372,738; 6,340,701; 5,846,545 and 5,585,499.

An attractive candidate for the preparation of such cytotoxic conjugates is CC-1065, which is a potent anti-tumor antibiotic isolated from the culture broth of Streptomyces zelensis. CC-1065 is about 1000-fold more potent in vitro than are commonly used anti-cancer drugs, such as doxorubicin, methotrexate and vincristine (B. K. Bhuyan et al., Cancer Res., 42, 3532-3537 (1982)).

Cytotoxic drugs such as methotrexate, daunorubicin, doxorubicin, vincristine, vinblastine, melphalan, mitomycin C, chlorambucil, and calicheamicin are also suitable for the preparation of conjugates of the present invention, and the drug molecules can also be linked to the antibody molecules through an intermediary carrier molecule such as serum albumin.

For diagnostic applications, the antibodies of the present invention typically will be labeled with a detectable moiety. The detectable moiety can be any one which is capable of producing, either directly or indirectly, a detectable signal. For example, the detectable moiety may be a radioisotope, such as H3, C14 or 13, P32, S35, or I131; a fluorescent or chemiluminescent compound, such as fluorescein isothiocyanate, rhodamine, or luciferin; or an enzyme, such as alkaline phosphatase, beta-galactosidase or horseradish peroxidase.

### Conjugation

Any method known in the art for conjugating the a protein to the detectable moiety may be employed, including those methods described by Hunter, et al., Nature 144:945 (1962); David, et al., Biochemistry 13:1014 (1974); Pain, et al., J. Immunol. Meth. 40:219 (1981); and Nygren, J. Histochem. and Cytochem. 30:407 (1982). In vitro methods, include conjugation chemistry well know in the art including chemistry compatible with proteins, such as chemistry for specific amino acids, such as Cys and Lys. In order to link a moiety (such as PEG) to a protein of the invention, a linking group or reactive group is used. Suitable linking groups are well known in the art and include disulfide groups, thioether groups, acid labile groups, photolabile groups, peptidase labile groups and esterase labile groups. Preferred linking groups are disulfide groups and thioether groups depending on the application. For fibronectin based scaffolds or other proteins with out a cys amino acid, a cys can be engineered in a location to allow for activity of the protein to exist while creating a location for conjugation.

### Exemplary Uses

The VEGFR-2 binding proteins described herein and their related variants are useful in a number of therapeutic and diagnostic applications. These include the inhibition of the biological activity of VEGF by competing for or blocking the binding to a VEGFR-2 as well as the delivery of cytotoxic or imaging moieties to cells, preferably cells expressing VEGFR-2.

The small size and stable structure of these molecules can be particularly valuable with respect to manufacturing of the drug, rapid clearance from the body for certain applications where rapid clearance is desired or formulation into novel delivery systems that are suitable or improved using a molecule with such characteristics.

On the basis of their efficacy as inhibitors of VEGF biological activity, the polypeptides of the invention are effective against a number of conditions associated with inappropriate angiogenesis, including but not limited to autoimmune disorders (e.g., rheumatoid arthritis, inflammatory bowel disease or psoriasis); cardiac disorders (e.g., atherosclerosis or blood vessel restenosis); retinopathies (e.g., proliferative retinopathies generally, diabetic retinopathy, age-related macular degeneration or neovascular glaucoma), renal disease (e.g., diabetic nephropathy, malignant nephrosclerosis, thrombotic microangiopathy syndromes; transplant rejection; inflammatory renal disease; glomerulonephritis; mesangioproliferative glomerulonephritis; haemolytic-uraemic syndrome; and hypertensive nephrosclerosis); hemangioblastoma; hemangiomas; thyroid hyperplasias; tissue transplantations; chronic inflammation; Meigs's syndrome; pericardial effusion; pleural effusion; autoimmune diseases; diabetes; endometriosis; chronic asthma; undesirable fibrosis (particularly hepatic fibrosis) and cancer, as well as complications arising from cancer, such as pleural effusion and ascites. Preferably, the VEGFR-binding polypeptides of the invention can be used for the treatment of prevention of hyperproliferative diseases or cancer and the metastatic spread of cancers. Non-limiting examples of cancers include bladder, blood, bone, brain, breast, cartilage, colon kidney, liver, lung, lymph node, nervous tissue, ovary, pancreatic, prostate, skeletal muscle, skin, spinal cord, spleen, stomach, testes, thymus, thyroid, trachea, urogenital tract, ureter, urethra, uterus, or vaginal cancer. Additional treatable conditions can be found in U.S. Patent No. 6,524,583. Other references describing uses for VEGFR-2 binding polypeptides include: McLeod DS et al., Invest Ophthalmol Vis Sci. 2002 Feb;43(2):474-82; Watanabe et al. Exp Dermatol. 2004 Nov;13(11):671-81; Yoshiji H et al., Gut. 2003 Sep;52(9):1347-54; Verheul et al., Oncologist. 2000;5 Suppl 1:45-50; Boldicke et al., Stem Cells. 2001;19(1):24-36.

As described herein, angiogenesis-associated diseases include, but are not limited to, angiogenesis-dependent cancer, including, for example, solid tumors, blood born tumors such as leukemias, and tumor metastases; benign tumors, for example hemangiomas, acoustic neuromas, neurofibromas, trachomas, and pyogenic granulomas; inflammatory disorders such as immune and non-immune inflammation; chronic articular rheumatism and psoriasis; ocular angiogenic diseases, for example, diabetic retinopathy, retinopathy of prematurity, macular degeneration, corneal graft rejection, neovascular glaucoma, retrolental fibroplasia, rubeosis; Osler-Webber Syndrome; myocardial angiogenesis; plaque neovascularization; telangiectasia; hemophiliac joints; angiofibroma; and wound granulation and wound healing; telangiectasia psoriasis scleroderma, pyogenic granuloma, cororany collaterals, ischemic limb angiogenesis, corneal diseases, rubeosis, arthritis, diabetic neovascularization, fractures, vasculogenesis, hematopoiesis.

### Additional Agents That May be Used with Appropriate Embodiments of the Invention

In other therapeutic treatments or compositions, the proteins of the invention are co-administered, or administered sequentially, with one or more additional therapeutic agents. Suitable therapeutic agents include, but are not limited to, targeted therapeutics, other targeted biologics, and cytotoxic or cytostatic agents. In some instances in will be preferred to administer agents from the same or separate therapeutically acceptable vial, syringe or other administration device that holds a liquid formulation.

Cancer therapeutic agents are those agents that seek to kill or limit the growth of cancer cells while having minimal effects on the patient. Thus, such agents may exploit any difference in cancer cell properties (e.g., metabolism, vascularization or cell-surface antigen presentation) from healthy host cells. Differences in tumor morphology are potential sites for intervention: for example, the second therapeutic can be an antibody such as an anti-VEGF antibody that is useful in retarding the vascularization of the interior of a solid tumor, thereby slowing its growth rate. Other therapeutic agents include, but are not limited to, adjuncts such as granisetron HCl, androgen inhibitors such as leuprolide acetate, antibiotics such as doxorubicin, antiestrogens such as tamoxifen, antimetabolites such as interferon alpha-2a, cytotoxic agents such as taxol, enzyme inhibitors such as ras farnesyl-transferase inhibitor, immunomodulators such as aldesleukin, and nitrogen mustard derivatives such as melphalan HCl, and the like.

The therapeutic agents that can be combined with proteins of the invention for improved anti-cancer efficacy include diverse agents used in oncology practice (Reference: Cancer, Principles & Practice of Oncology, DeVita, V. T., Hellman, S., Rosenberg, S. A., 6th edition, Lippincott-Raven, Philadelphia, 2001), such as docetaxel, paclitaxel, doxorubicin, epirubicin, cyclophosphamide, trastuzumab, capecitabine, tamoxifen, toremifene, letrozole, anastrozole, fulvestrant, exemestane, goserelin, oxaliplatin, carboplatin, cisplatin, dexamethasone, antide, bevacizumab, 5-fluorouracil, leucovorin, levamisole, irinotecan, etoposide, topotecan, gemcitabine, vinorelbine, estramustine, mitoxantrone, abarelix, zoledronate, streptozocin, rituximab, idarubicin, busulfan, chlorambucil, fludarabine, imatinib, cytarabine, ibritumomab, tositumomab, interferon alpha-2b, melphalam, bortezomib, altretamine, asparaginase, gefitinib, erlonitib, anti-EGF receptor antibody (e.g., cetuximab or panitumab), ixabepilone, epothilones or derivatives thereof, and conjugates of cytotoxic drugs and antibodies against cell-surface receptors. Preferred therapeutic agents are platinum agents (such as carboplatin, oxaliplatin, cisplatin), taxanes (such as paclitaxel, docetaxel), gemcitabine, and camptothecin.

The one or more additional therapeutic agents can be administered before, concurrently, or after the antibody, antibody fragment or conjugate of the invention. The skilled artisan will understand that for each therapeutic agent there may be advantages to a particular order of administration. Similarly, the skilled artisan will understand that for each therapeutic agent, the length of time between which the agent, and an antibody, antibody fragment or conjugate of the invention is administered, will vary.

### Formulation and Administration

Therapeutic formulations of the invention are prepared for storage by mixing the described proteins having the desired degree of purity with optional physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyidimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN ^{™}, PLURONICS^{™} or polyethylene glycol (PEG).

The formulations herein may also contain more than one active compound as necessary for the particular indication being treated, preferably those with complementary activities that do not adversely affect each other. Examples of combinations of active compounds are provided in herein. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes.

Sustained-release preparations may be prepared. Suitable examples of sustained-release preparations include semipermeable matrices of solid hydrophobic polymers containing the proteins of the invention, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and y ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated proteins of the invention may remain in the body for a long time, they may denature or aggregate as a result of exposure to moisture at 37C, resulting in a loss of biological activity and possible changes in immunogenicity. Rational strategies can be devised for stabilization depending on the mechanism involved. For example, if the aggregation mechanism is discovered to be intermolecular S--S bond formation through thiodisulfide interchange, stabilization may be achieved by modifying sulfhydryl residues, lyophilizing from acidic solutions, controlling moisture content, using appropriate additives, and developing specific polymer matrix compositions.

Suitable pharmaceutically acceptable carriers, diluents, and excipients are well known and can be determined by those of skill in the art as the clinical situation warrants. Examples of suitable carriers, diluents and/or excipients include: (1) Dulbecco's phosphate buffered saline, pH about 7.4, containing about 1 mg/ml to 25 mg/ml human serum albumin, (2) 0.9% saline (0.9% w/v NaCl, and (3) 5% (w/v) dextrose.

In some embodiments, the formulation comprises 5-100 mM sodium acetate, mannitol or a comparable excipient (e.g., sorbitol) at concentrations from 50 mM to and including hypertonic concentrations, optionally sodium chloride from 0 to around 200 mM, with a pH from around 4 to around 7.5. In some embodiments, the formulation comprises 5-100 mM sodium acetate, 0 to around 200 mM sodium chloride, and 50-150 mM mannitol, wherein the pH is from around 4.5 to around 6.0. In one embodiment, the formulation comprises around 10 mM sodium acetate, around 100 mM sodium chloride, and around 110 mM mannitol with a pH of around 4.5. In another embodiment, the formulation comprises around 10 mM sodium acetate and around 100 mM of mannitol at a pH from around 4.5 to 6. The protein of the invention will be formulated at a concentration of about 0.1 mg/ml to 100 mg/ml. In one embodiment, the concentration of the protein is from 1 to 15 mg/ml. In one embodiment the concentration of the protein is from around 9 to around 11 mg/ml.

Depending on the type and severity of the disease, preferably from about 1 mg/square meter to about 2000 mg/square meter of protein is an initial candidate dosage for administration to the patient, more preferably from about 10 mg/square meter to about 1000 mg/square meter of antibody whether, for example, by one or more separate administrations, or by continuous infusion. For repeated administrations over several days or longer, depending on the condition, the treatment is repeated until a desired suppression of disease symptoms occurs. However, other dosage regimens may be useful and are not excluded.

For therapeutic applications, the proteins or conjugates of the invention are administered to a subject, in a pharmaceutically acceptable dosage form. They can be administered intravenously as a bolus or by continuous infusion over a period of time, by intramuscular, subcutaneous, intra-articular, intrasynovial, intrathecal, oral, topical, or inhalation routes. The protein may also be administered by intratumoral, peritumoral, intralesional, or perilesional routes, to exert local as well as systemic therapeutic effects. One skilled in the art will recognize that the appropriate dosage of a protein of the invention depends on many factors include the bioavailability.

In some embodiments, the proteins of the invention are administered intravenously to a subject having a condition associated with inappropriate angiogenesis. In some embodiments, the protein is administered in a dosage between about 0.5 to about 4 mg/kg. In some embodiments the protein is administered at a dosage of about 1 mg/kg or about 3 mg/kg. The protein may be administered at least once per month, once per week, twice per week, or once per day.

In some embodiments, the proteins of the invention are subcutaneously administered. The proteins are formulated into pharmaceutically acceptable compositions and may be administered twice daily, once daily, on alternative days, or weekly. In some embodiments, the proteins are administered between 0.5 mg/kg to 2 mg/kg or between around 0.1 to around 0.3 mg/kg. In some embodiments, the proteins are administered at 0.1, 0.2, 0.3, or 0.4 mg/kg daily. In some embodiments, the patient is first administered an intravenous load of protein, for example from 0.5 to 2mg/kg, and is subsequently administered the protein subcutaneously.

The dosage and dosing schedule of the polypeptide may be adjusted based on the plasma concentration of polypeptide to be achieved in the subject. According to the invention, administration of the polypeptide results in a peak concentration of between 1 and 7 µM.

The dosage and dosing schedule of the polypeptide may be adjusted based on the plasma concentration of VEGF-A to be achieved in the subject. For example, in some embodiments, administration of the polypeptide results in a peak concentration of between 5 and 40 pM, between 10 and 30 pM, between 10 and 25 pM, or between 10 and 20 pM.

The present invention also includes kits comprising one or more of the elements described herein, and instructions for the use of those elements. In a preferred embodiment, a kit of the present invention includes a polypeptide of the invention and a therapeutic agent. The instructions for this preferred embodiment include instructions for inhibiting the growth of a cancer cell using the protein of the invention, and the therapeutic agent, and/or instructions for a method of treating a patient having a cancer using the antibody, antibody fragment or conjugate of the invention, and the therapeutic agent.

Preferably, the therapeutic agent used in the kit is selected from the group consisting of docetaxel, paclitaxel, doxorubicin, epirubicin, cyclophosphamide, trastuzumab, capecitabine, tamoxifen, toremifene, letrozole, anastrozole, fulvestrant, exemestane, goserelin, oxaliplatin, carboplatin, cisplatin, dexamethasone, antide, bevacizumab, 5-fluorouracil, leucovorin, levamisole, irinotecan, etoposide, topotecan, gemcitabine, vinorelbine, estramustine, mitoxantrone, abarelix, zoledronate, streptozocin, rituximab, idarubicin, busulfan, chlorambucil, fludarabine, imatinib, cytarabine, ibritumomab, tositumomab, interferon alpha-2b, melphalam, bortezomib, altretamine, asparaginase, gefitinib, erlonitib, anti-EGF receptor antibody (e.g., cetuximab or panitumumab), ixabepilone, and an epothilone or derivative thereof. More preferably, the therapeutic agent is a platinum agent (such as carboplatin, oxaliplatin, cisplatin), a taxane (such as paclitaxel, docetaxel), gemcitabine, or camptothecin.

The elements of the kits of the present invention are in a suitable form for a kit, such as a solution or lyophilized powder. The concentration or amount of the elements of the kits will be understood by the skilled artisan to varying depending on the identity and intended use of each element of the kit.

When a kit is supplied, the different components of the composition may be packaged in separate containers and admixed immediately before use. Such packaging of the components separately may permit long-term storage without losing the active components' functions.

The reagents included in the kits can be supplied in containers of any sort such that the life of the different components are preserved and are not adsorbed or altered by the materials of the container. For example, sealed glass ampoules may contain lyophilized therapeutic agents, or buffers that have been packaged under a neutral, non-reacting gas, such as nitrogen. Ampoules may consist of any suitable material, such as glass, organic polymers, such as polycarbonate, polystyrene, etc., ceramic, metal or any other material typically employed to hold similar reagents. Other examples of suitable containers include simple bottles that may be fabricated from similar substances as ampoules, and envelopes, that may comprise foil-lined interiors, such as aluminum or an alloy. Other containers include test tubes, vials, flasks, bottles, IV bags, syringes, or the like. Containers may have a sterile access port, such as a bottle having a stopper that can be pierced by a hypodermic injection needle. Other containers may have two compartments that are separated by a readily removable membrane that upon removal permits the components to be mixed. Removable membranes may be glass, plastic, rubber, etc.

Kits may also be supplied with instructional materials. Instructions may be printed on paper or other substrate, and/or may be supplied as an electronic-readable medium, such as a floppy disc, CD-ROM, DVD-ROM, Zip disc, videotape, audiotape, flash memory device etc. Detailed instructions may not be physically associated with the kit; instead, a user may be directed to an internet web site specified by the manufacturer or distributor of the kit, or supplied as electronic mail.

The cancers and cells there from referred to in the instructions of the kits include breast cancer, colon cancer, ovarian carcinoma, osteosarcoma, cervical cancer, prostate cancer, lung cancer, synovial carcinoma, pancreatic cancer, melanoma, multiple myeloma, neuroblastoma, and rhabdomyosarcoma.

The dosage of cytotoxic or therapeutic agents administered in the methods described herein can be readily determined by those skilled in the art. Pharmaceutical package inserts may also be consulted when determining the proper dosage. By way of example, the package inserts for Sutent^{™}; Nexavar^{™}; bevacizumab; trastuzumab; gemcitabine; temozolomide; Gleevac^{™}; paclitaxel; and doxetaxel are referred to.

One aspect of the invention provides for the use of plasma VEGF-A concentrations to determine an effective dosage of the proteins to be administered. Plasma VEGF-A levels have been linked as a marker for VEGFR2 inhibition by anti-VEGFR2 antibodies (Bocci, et al. Cancer Res. 64: 6616-6625 (2004))

VEGF-A plasma concentrations may be determined in a subject by a variety of known assays. Immunoassays are commonly used to quantitate the levels of proteins in cell samples, and many other immunoassay techniques are known in the art. Exemplary immunoassays which may be conducted according to the invention include fluorescence polarization immunoassay (FPIA), fluorescence immunoassay (FIA), enzyme immunoassay (EIA), nephelometric inhibition immunoassay (NIA), enzyme-linked immunosorbent assay (ELISA), and radioimmunoassay (RIA). An indicator moiety, or label group, may be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

A baseline VEGF-A plasma concentration may be determined in a subject prior to the onset of polypeptide therapy. The baseline may also be determined between polypeptide administration, e.g., at the polypeptide trough level. The baseline may also be determined from a reference value, such as from the average plasma VEGF-A concentration in a population.

The VEGF-A plasma concentration is determined in a subject after administration of a protein of the invention. The plasma concentration may be determined after around 2, 4, 6, 8, 10, 12, 18, 24, 36, or 48 hours or after several days or more after administration of a protein of the invention and the concentration determined may be the VEGF-A peak plasma concentration. The dosage administered to the subject is adjusted to achieve at least a 20, 30, 40, 50, 60, 70, 80, 90% or more increase in plasma VEGF-A concentration over baseline concentration. The dosage administered to the subject may be adjusted to achieve a maximal increase in plasma VEGF-A concentration in the subject. A person skilled in the art will appreciate that the maximum plasma VEGF-A concentration will vary among subjects. While not wishing to be bound by theory, it is believed that plasma VEGF-A concentrations are an indirect measurement of VEGFR2 occupancy. Therefore, the greater the increase in VEGF-A concentration, the more VEGFR2 receptors are bound to the proteins of the invention and consequently the higher the inhibition of VEGFR2 inhibition.

Another aspect of the invention provides that the efficacy of a protein of the invention in treating a subject having a condition associated with inappropriate angiogenesis may be determined by a subject's VEGF-A plasma concentration. The plasma concentration of VEGF-A in a subject is determined after administration of a protein of the invention. The plasma concentration may be determined after around 2, 4, 6, 8, 10, 12, 18, 24, 36, or 48 hours or after several days or more after administration of a protein of the invention and the concentration determined may be the VEGF-A peak plasma concentration. The efficacy of the treatment is determined based on the change of VEGF-A levels in the subject over the baseline. A large increase in plasma VEGF-A concentration relative to the baseline indicates a good prognosis for treatment.

Another aspect of the invention provides methods for monitoring the risk of toxicity based on VEGF-A plasma concentration. The plasma concentration of VEGF-A is determined in a patient after administration of a polypeptide of the invention. The plasma concentration may be determined after 2, 4, 6, 8, 10, 12, 18, 24, 36, or 48 hours or after several days or more after administration of a protein of the invention and the concentration determined may be the VEGF-A peak plasma concentration. The subject's plasma VEGF-A concentration is then compared to a VEGF-A toxicity risk concentration. A toxicity risk concentration may be at least 2, 3, 4, 5, 6, 7, 8, 9, 10, 15, 20, 30, 40, 50 pM or more of plasma VEGF-A concentration. A toxicity risk concentration can also be calculated based on a subject's baseline plasma VEGF-A concentration determined before polypeptide therapy was initiated. The toxicity risk concentration can be at least 2, 3, 4, 5, 6, 7, 8, 9, 10, 15, 20, 30 times or more the subject's baseline plasma VEGF-A concentration. The toxicity risk can also be determined from the average plasma VEGF-A concentrations that induce dose limiting toxicities in a population. A skilled person is able to evaluate the toxicity risk as one of many factors affecting patient treatment.

Another aspect of the invention provides methods for monitoring an immunogenic response to a protein of the invention. A polypeptide is administered to a subject followed by the determination of the VEGF-A plasma concentration in the subject. The plasma concentration may be determined after 2, 4, 6, 8, 10, 12, 18, 24, 36, or 48 hours or after several days or more after administration of a protein of the invention and the concentration determined may be the VEGF-A peak plasma concentration. The subject's VEGF-A plasma concentration is then compared to the VEGF-A plasma concentration determined at one or more prior time points. The VEGF-A plasma concentration previously determined may be a peak concentration. The prior time points include a point after a prior polypeptide administration. The concentration determined at one or more prior time points includes an average of the VEGF-a plasma concentration determined at two or more prior time points.

While not wishing to be bound by theory, a subject may begin to produce neutralizing antibodies that bind to a protein of the invention preventing the binding to VEGFR2. As a result, VEGF-A plasma concentrations decrease or fail to increase after polypeptide administration to the same extent as in previous administrations.

### EXAMPLES

The invention is now described by reference to the following examples, which are illustrative only, and are not intended to limit the present invention. While the invention has been described in detail and with reference to specific embodiments thereof, it will be apparent to one of skill in the art that various changes and modifications can be made thereto without departing from the spirit and scope thereof.

The examples below were performed with Comp-I, an exemplary fibronectin based domain scaffold VEGFR2 binder.

### Example 1: Murine Pro-B Cell line Proliferation Assay

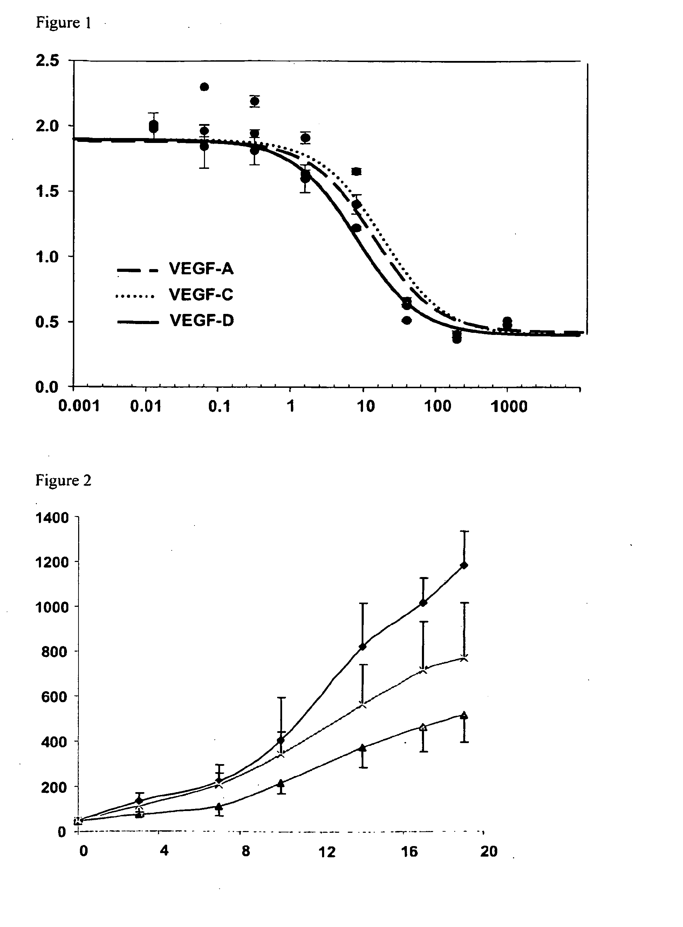

Murine Ba/F3 cells stably expressing a VEGFR2 fusion protein (comprising the extracellular domain of hVEGFR2 and the intracellular domain of hEpoR) were plated in 96-well plates at 25,000 cells/well in 90 mL growth media containing 15 ng/mL of VEGF-A, VEGF-C, or VEGF-D. Serial dilutions of Comp-I were prepared at 10x final concentration, and 10 mL of the Comp-I was added to each well. Plates were incubated at 37°C/5% CO2 for 48-72 hours. Cell proliferation assay reagent (CellTiter 96, Promega) was added to each well (20 mL/well), and the plates were further incubated for 3-4 hours. At the end of the incubation period, absorbance was read (A490) in a 96-well platereader. Figure 1 depicts the results of Comp-I inhibition on VEGF-A, VEGF-C, and VEGF-D mediated proliferation.

### Example 2: Tumor inhibition

Comp-I demonstrates tumor inhibition in a broad range of mouse and human tumor models, as shown in Table 1.

**Table 1.**

| Cell line | Tissue | Tumor of Origin | Species | Model | Inhibition |
|---|---|---|---|---|---|
| B16-F10 | Skin | Melanoma | Mouse | Syngeneic s.c | √ |
| CT26 | Colon | Carcinoma | Mouse | Syngeneic s.c. | √ |
| A2058 | Skin | Melanoma | Human | Xenogeneic s.c. | √ |
| Colo-205 | Colon | Carcinoma | Human | Xenogeneic s.c. | √ |
| U87 | Brain | Glioblastoma | Human | Xenogeneic s.c. | √ |
| A549 | Lung | NSCLC | Human | Xenogeneic s.c. | √ |
| | | Adenocarcinoma | | | |
| Mia2 | Pancreas | Carcinoma | Human | Orthotopic | √ |

The effect of Comp-I and bevacizumab treatment was assessed in a U87 xenograft model of human glioblastoma (Figure 2). Briefly, nude male mice, 6-7 weeks of age, were implanted with U87 glioblastoma tumor cells (5 x 10⁶) subcutaneously on the right flank. Tumor growth was monitored 2 times per week using caliper measurement. Drug treatment was initiated after the tumors reached a size of at least 50 mm³. Tumor growth inhibition over vehicle was greater in Comp-I (56%) as compared to bevacizumab (35%) treatment.

The anti-tumor effect of Comp-I was compared to bevacizumab treatment in a model of colon (Colo205 colon carcinoma) and brain (U87 glioblastoma) tumors. Comp-I demonstrated a 57% greater effect on tumor reduction in the colon tumor model and a 59% greater effect on tumor reduction in the brain tumor model as compared to bevacizumab treatment.

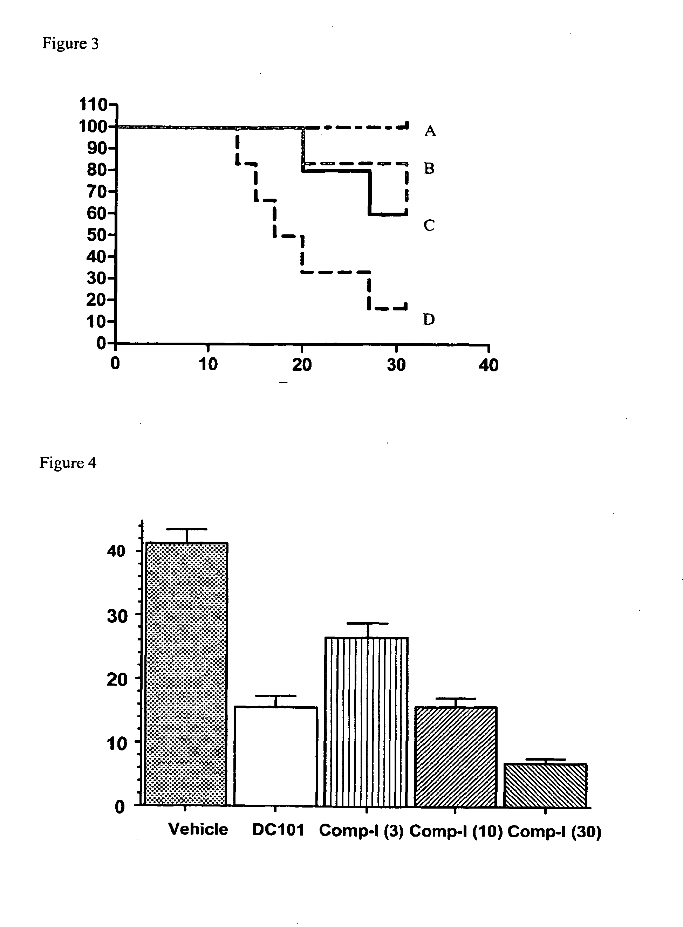

The anti-tumor effect of Comp-I was also compared to treatment with sunitinib and sorafenib in a Colo205 colon carcinoma model. Figure 3 depicts a Kaplan Meier Survival analysis on the length of time for individual tumors to reach a given size. In this experiment, treatments were initiated when tumors reached an average size of 200 mm³, and the graph scores the time it takes for each individual tumor to reach 500 mm³ Comp-I treatment resulted in 100% of tumors remaining under 500 mm³, while only around 60% of tumors remained under 500 mm³ with sunitinib or sorafenib treatment. All three compounds were administered at 60 mg/kg.

An additional study was performed using the Colo205 colon carcinoma model to test the toxicity of sunitinib (60 mg/kg/day), sorafenib (80 mg/kg/day), and Comp-I (30 mg/kg/day) treatment. No toxicity was observed in 15/15 mice treated with Comp-I. However, sunitinib treated mice demonstrated a loss in muscle and fat and only 12/15 mice survived. Sorafenib treated mice demonstrated skin scaling and rash and 14/15 mice survived. These results suggest that the inhibition of multiple kinases (by sorafenib and sunitinib) may cause more toxicity than mono-specific VEGFR2 blockers (come-I) at dosage levels resulting in similar efficacy.

### Example 3: Blood Vessel Density

The effect of Comp-I treatment on microvascular density was assessed in a U87 human glioblastoma mouse xenograft model. (Figure 4). Histological analysis of tumors excised post-treatment showed that inhibition of growth by Comp-I was associated with a greatly reduced microvessel density and was comparably efficacious to DC101, the anti-mouse VEGFR2 monoclonal antibody.

### Example 4: Blood Pressure effects

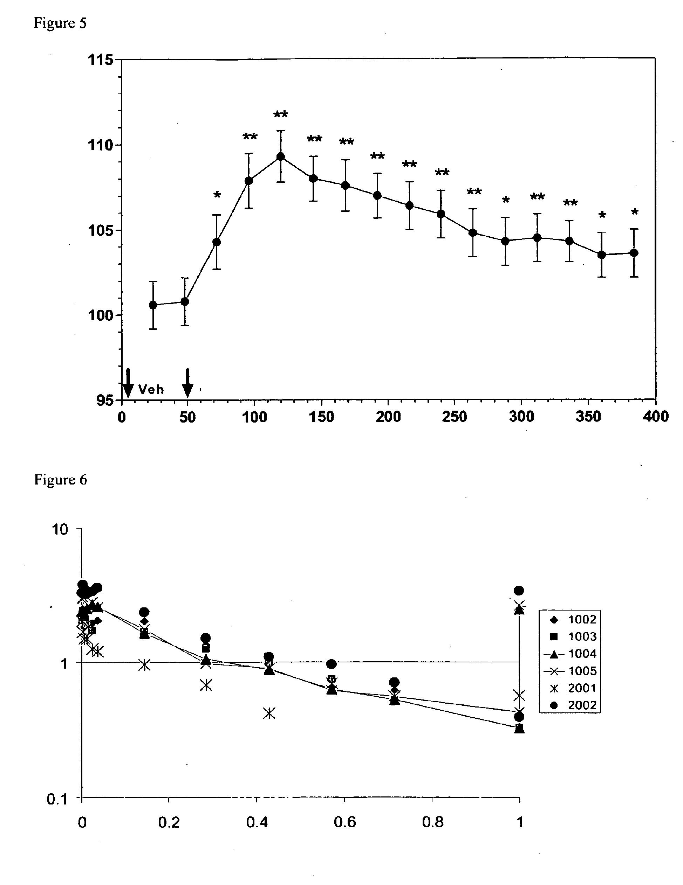

The effect of Comp-I treatment on blood pressure was determined in rats (Figure 5). Male Sprague-Dawley rats (250 g) were housed singly in cages maintained in a controlled temperature (22-25° C) and humidity (60-70%) environment with a 12 hour light-dark cycle and were provided standardized rodent chow (Purina 5001) and water ad libum. Animals were anesthetized with pentobarbital sodium (50 mg/kg ip) and were surgically implanted with PhysiotelTM C50-PXT blood pressure telemetry transmitter devices (Data Sciences International, St. Paul, MN) by inserting and securing the pressure catheter tip of the transmitter into the abdominal aorta. Instrumented animals were allowed to recover for at least one week following surgery. For the study, vehicle was first administered by intravenous injection and mean arterial blood pressure (MAP) was recorded every 30 minutes for 48 hours in order to establish a baseline. Comp-I (50 mg/kg) was then administered into the same animals by intravenous injection and blood pressure was recorded every 30 minutes for the initial 48 hours after treatment. Thereafter, blood pressure was recorded every hour for 5 days (day 7 post-treatment) and then every 4 hours for another 7 days (day 14 post-treatment). All data points represent the mean of all blood pressure measurements over a 24 hour period ± SEM.

### Example 5: Phase I Study

A phase I study was conducted to determine the safety, tolerability, maximum tolerated dose (MTD), pharmacokinetics (PK), and biomarker response (PD) associated with administration of Comp-I. Patients with solid tumor or non-Hodgkin's lymphoma and no standard treatment option; ECOG 0-2; and adequate organ function were included. Twelve patients were enrolled for two weekly dosing cohorts. Patients 1002-1005, 2001, and 2002 received 1 mg/kg/week of Comp-I. Patients 1006, 1008, 1009, 2003-2005 received 3 mg/kg/week of Comp-I. Ages ranged from 31 to 79 with a median age of 57. Cancer types included hormone refractory prostate carcinoma (3), colon carcinoma (2), thymoma (1), metastatic neuroendocrine carcinoma (1), kidney transitional cell carcinoma (1), anal squamous cell carcinoma (1), endometrial carcinoma (1), signet ring cell carcinoma (1), and adenocystic carcinoma (1). Eleven subjects had prior chemotherapy, 6 subjects had prior radiotherapy, 4 subjects had prior hormonal therapy, 6 subjects had prior surgery, and 2 patients were previously treated with bevacizumab.

The MTD was 2 mg/kg/wk. Dose limiting toxicities included Gr4 lipase elevation (1; 1 mg/kg QW); Gr3 proteinuria (2; 3 mg/kg QW); Gr3 LV dysfunction (1; 3 mg/kg QW). Additionally, there was reversible Gr1 hypertension (1; mg/kg QW), reversible Gr1 proteinuria (1; 1 mg/kg QW), and reversible Gr2 proteinuria (1; 1 mg/kg QW). There were no acute infusion reactions or clinically significant immunogenicity.

While conventionally viewed as side effects, both hypertension and proteinuria may also be used as markers for the inhibition of VEGFR signaling (van Heeckeren, WJ et al., J Clinical Oncol 25:2993-2995 (2007). The presence of these markers in the phase I subjects indicates the in vivo efficacy of VEGFR signaling inhibition in humans.

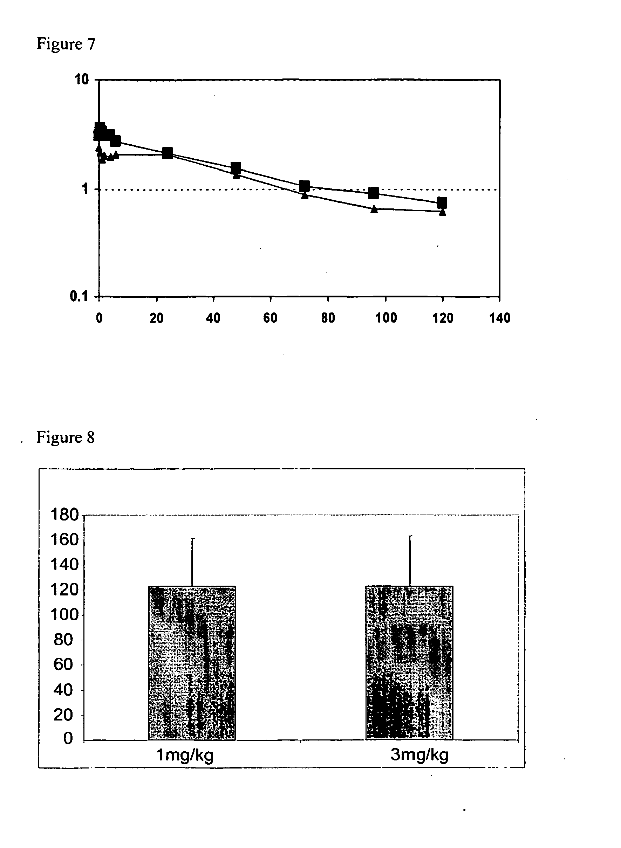

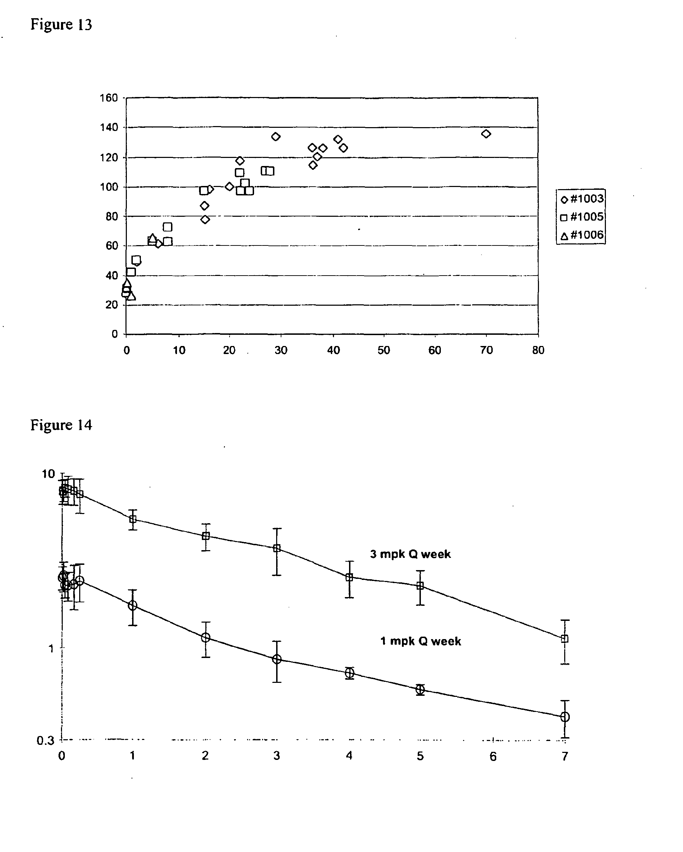

Figure 6 demonstrates that the first dose pharmokinetic profile of Comp-I is similar among subjects. Note that the spike in Comp-I concentration is the peak of the second dose administered. Drug concentrations were measured with an enzyme-linked immunosorbent assay (ELISA) where drug was captured from plasma with an anti-PEG monoclonal antibody and the immobilized drug was detected with a biotinylated VEGFR-2 protein fusion with human IgG Fc domain, followed by a horse-radish peroxidase-conjugated streptavidin mixture. Figure 7 demonstrates that the pharmacokinetic profile is similar after 6 months of 1 mg/kg weekly Comp-I treatment (Patient 1003). Figure 8 demonstrates that trough levels of Comp-I are consistent over time in both treatment cohorts. Figure 14 and Table 2 depict the phamacokinetics of 1 mg/kg and 3 mg/kg weekly Comp-I treatment.

**Table 2.**

| | 1mg/kg QW | 3 mg/kg QW |
|---|---|---|
| | (n = 6) | (n = 6) |
| Cmax (uM/L) mean (range) | 2.7 (2.0-3.8) | 8.7 (7.2-10.8) |
| AUC (uM*day) mean (range) | 9.1 (8.0-10.6) | 29.1 (25.4-33.8) |
| T1/2 (h) median (range) | 68.7 (56.9 -157.2) | 61.1 (49.4 - 89.3) |
| CL (L/daylkg) mean (SD) | 0.011 (0.001) | 0.010 (0.001) |

### Example 6: VEGF-A as a biomarker

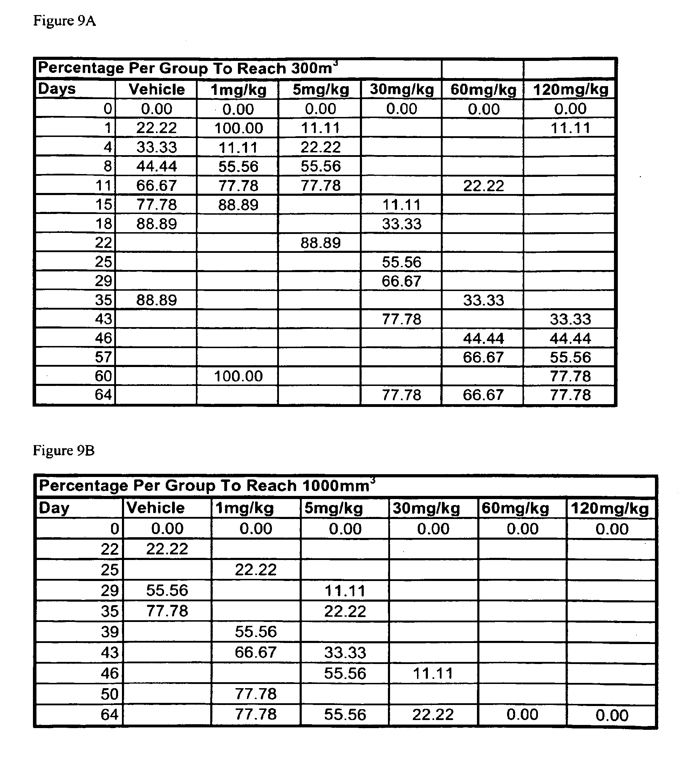

The Colo225 model was utilitized to optimize Comp-I dosing and to measure biomarkers of activity. Tumor cells were implanted in female NCRNU-M mice and drug treatment initiated after the tumors reached a size of at least 50 mm³ (Figures 9A and 9B). Comp-I was administered at 1, 5, 30, 60, and 120 mg/kg three times a week. Percent survival was defined as those animals surviving with tumors smaller that the noted cut-off (300 and 1000 mm³). Intra-peritoneal administration of Comp-I at 60 mg/kg three times weekly resulted in the maximal anti-tumor activity for this drug. Administration of 120 mg/kg did not improve tumor suppression as measured by the stringent cut-off of time for tumors to reach 300 mm³. Analyzing results with a less stringent cut-off of 1000 mm³ indicated that doses as low as 5 mg/kg have substantial efficacy compared to vehicle control groups. Furthermore, while 30 mg/kg dose was not maximally efficacious, it was highly effective compared to vehicle and 1 or 5 mg/kg doses.

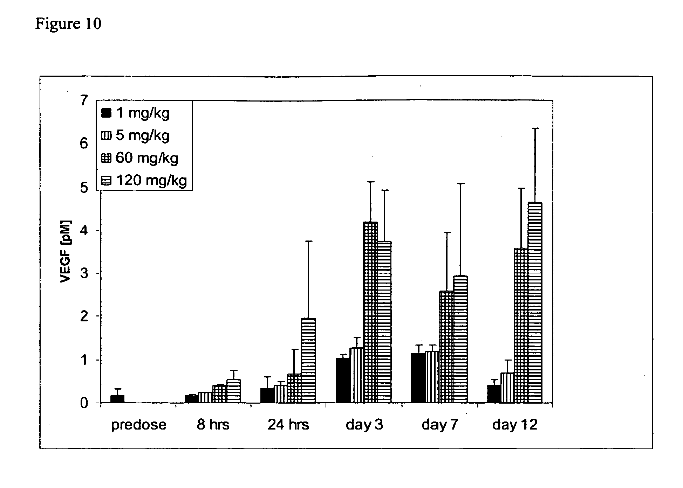

A similar dosing schedule was used for up to 12 days in tumor-free mice of the same strain used in the Colo205 model above in order to establish Comp-I and VEGF-A concentrations as biological markers associated with efficacy in the tumor model (Figure 10). Comp-I induced a dose and time dependent increase in murine VEGF-A. For any given dose, VEGF-A increased to an approximate sustained maximum by 24 hours after beginning drug administration. The dose required for maximum induction of VEGF-A increase was 60 mg/kg three times weekly, analogous to the dose required for maximal tumor suppression of a Colo205 xenograft in the same strain of mice.

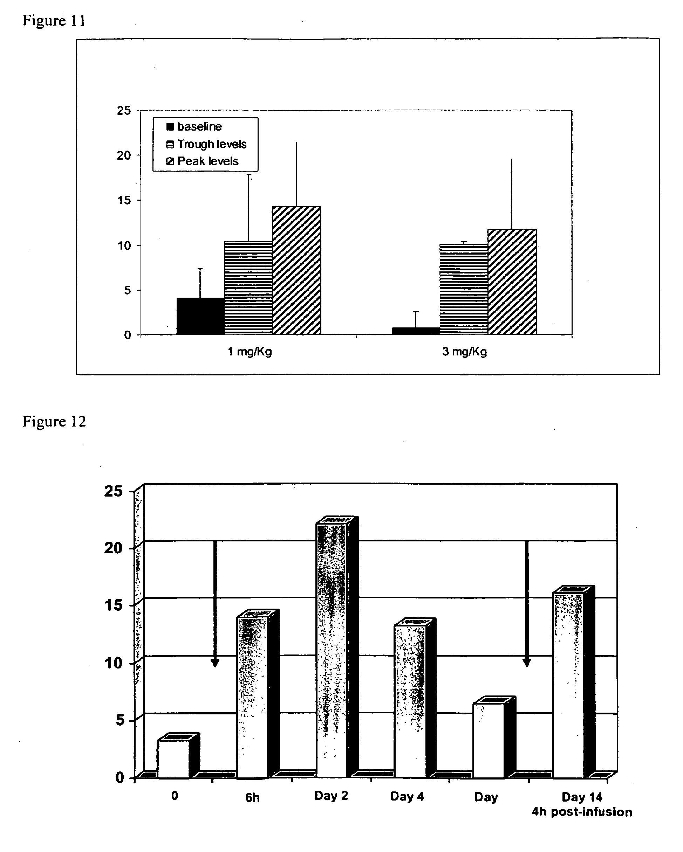

VEGF-A was also used as a biomarker response to Comp-I treatment in the Phase I study. Levels of human VEGF-A were assessed using a commercially available sandwich ELISA assay (R&D systems Inc) following the manufacturers specifications with the exception of the plasma dilution which was diluted 1:2. Pre-infusion plasma VEGF-A levels (trough) are greater than 70% of the four hour post-infusion (peak) levels following one course of weekly treatment (Figure 11). VEGF-A levels were measured in subject 1002 before and after Comp-I treatment (Figure 12). VEGF-A trough levels in subjects treated with 1 mg/kg per week of Comp-I remain elevated after 4 months of treatment (Table 3). Soluble VEGFR2 levels were also used as a biomarker response to Comp-I treatment (Figure 13). Levels of human soluble VEGFR-2 were assessed using a commercially available sandwich ELISA assay (R&D systems Inc) following the manufacturers specifications with the only difference that 1 uM Comp-I was spiked in the standard curve and samples to normalize for varying Comp-I levels in plasma samples.

**Table 3 VEGF-A (pM) in 6 subjects.**

| | 1002 | 1003 | 1004 | 1005 | 2001 | 2002 |
|---|---|---|---|---|---|---|
| 1 week | 12.02 | 18.77 | 5.95 | 3.56 | 16.9 | 11.46 |
| 3 | | | | | | |
| weeks | | 11.19 | 9.76 | 3.83 | 20.03 | 12.32 |
| 1 | | | | | | |
| month | | 17.6 | 5.35 | 5.9 | | |
| 2 | | | | | | |
| months | | 14.81 | | | | |
| 3 | | | | | | |
| months | | 9.99 | | | | |
| 4 | | | | | | |
| months | | 19.13 | | | | |

Results from the Phase I study demonstrate predictable pharmacokinetics of Comp-I administration, as well as predictable pharmacodynamics based on VEGF-A levels. Comp-I concentrations achieved in human plasma correlate with plasma exposure that was associated with mouse anti-tumor activity. Clinical observations of VEGFR-2 blockade including proteinuria and hypertension were also observed in both patient cohorts.

### Example 7: Subcutaneous Dosing Model

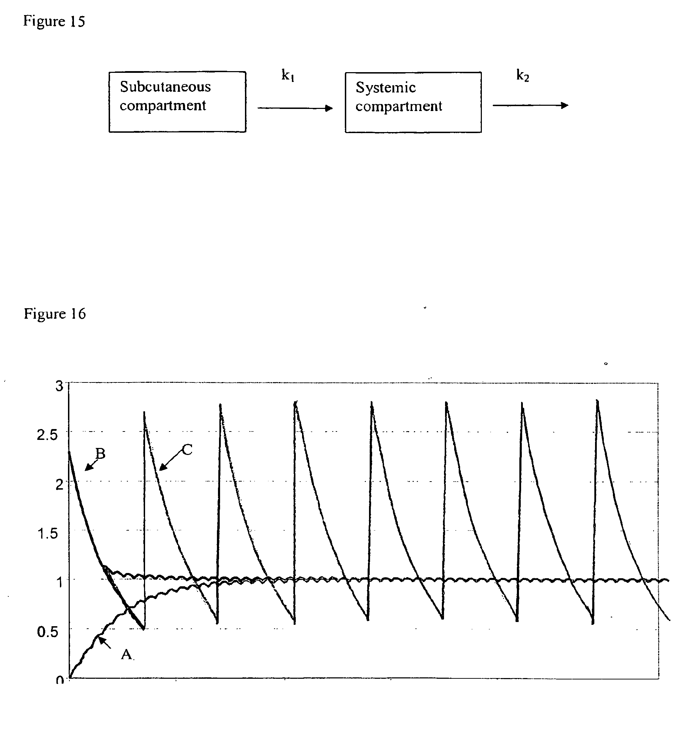

The administration of Comp-I was modeled using the program Berkeley Madonna (developed by Robert Macey and George Oster of the University of California at Berkeley) to identify magnitude of dose and frequency of subcutaneous administration required to reach potentially efficacious drug concentrations. The model is defined as a subcutaneous compartment that distributes with a first order process (k1) to a systemic compartment where the drug is subsequently eliminated by a first order process (k2, Figure 15). The subcutaneous compartment was assumed as approximately 1 ml that was diluted to approximately 5 L in the systemic compartment.

The absorption rate constant (k1) was assumed to be 1.4 day-1, which is in the range of pegylated interferon (Pegasys product insert) where Cmax is reached between 72 and 96 hours. The elimination rate constant (k2) was assumed to be 0.23 day-1, similar to the range for Comp-I in a phase 1 clinical study. The dose frequency was once per day. The multiple administrations were modeled using the "pulse" function of the program.

Using subcutaneous administration alone results in a slow accumulation to steady-state drug concentrations (Figure 16, "A"). A single intravenous bolus administration of Comp-I, with a first-order elimination rate constant of 0.23 day⁻¹, followed by subcutaneous administration of 0.1 mg/kg/day starting on Day 3 results in a faster accumulation to steady-state drug concentrations (Figure 16, "B"). The single intravenous bolus administration delivers rapid potentially efficacious systemic drug levels that are subsequently maintained by the subcutaneous administration.

## Claims

1. A polypeptide for use in treating a condition selected from an autoimmune disorder, an inflammatory disorder, a retinopathy, and a cancer, wherein the polypeptide comprises the amino acid sequence set forth in SEQ ID NO:4 and binds human VEGFR2 with a disassociation constant of 1 µM or less, wherein administration of the polypeptide results in a peak concentration of between 1 and 7 µM of the polypeptide.

## Patentansprüche

1. Polypeptid zur Verwendung in der Behandlung eines unter einer autoimmunen Erkrankung, einer entzündlichen Erkrankung, einer Retinopathie und einer Krebserkrankung ausgewählten Zustandes, wobei das Polypeptid die in SEQ ID NO:4 dargestellte Aminosäuresequenz umfasst und humanen VEGFR2 mit einer Dissoziationskonstante von 1 µM oder weniger bindet, wobei die Verabreichung des Polypeptids zu einer Maximalkonzentration von 1 bis 7 µM des Polypeptids führt.

## Revendications

1. Polypeptide en vue d'une utilisation dans le traitement d'un état sélectionné parmi un trouble auto-immune, un trouble inflammatoire, une rétinopathie et un cancer, dans lequel le polypeptide comprend la séquence d'acide aminé représentée dans la SEQ ID NO:4 et lie le VEGFR2 humain à une constante de dissociation de 1 µm ou moins, dans lequel l'administration du polypeptide résulte en une concentration maximale de 1 à 7 µm du polypeptide.
